# FASCICULE DE BREVET EUROPEEN

(11) **EP 2 790 692 B1**
(45) Date de publication et mention de la délivrance du brevet: **23.11.2016**
(21) Numéro de dépôt: 12808360.7
(22) Date de dépôt: 17.12.2012
(51) Int. Cl.: A61K 31/165, A61K 31/192, A61K 31/216, A61K 38/00, A61K 45/06, A61P 17/00, A61K 31/4178, A61K 31/454

(54) **ASSOCIATION D'UN AGONISTE DES RÉCEPTEURS DES PROSTAGLANDINES ET D'UN AGONISTE DU RÉCEPTEUR MC1R POUR LE TRAITEMENT ET/OU LA PRÉVENTION DE DÉSORDRES DE LA PIGMENTATION**
KOMBINATION VON EINEM PROSTAGLANDIN-REZEPTOR-AGONIST UND EINEM MC1R-REZEPTOR-AGONIST ZUR BEHANDLUNG UND/ODER VORBEUGUNG VON PIGMENTIERUNGSSTÖRUNGEN
ASSOCIATION OF A PROSTAGLANDIN RECEPTOR AGONIST AND OF A MC1R RECEPTOR AGONIST FOR TREATING AND/OR PREVENTING PIGMENTATION DISORDERS

(30) Priorité: 16.12.2011 FR 1161779
(43) Date de publication de la demande: 22.10.2014
(73) Titulaire: Galderma Research & Development, 06410 Biot (FR)
(72) Inventeur: MARTEL, Philippe, F-06410 Biot (FR); SUZUKI, Itaru, Izunokuni Shizuoka 410-2315 (JP); VOEGEL, Johannes, F-06740 Châteauneuf de Grasse (FR); ANDRES, Philippe, F-06530 Peymeinade (FR); RETHORE, Sandrine, F-06560 Valbonne (FR)
(74) Mandataire: Casalonga
(86) Numéro de dépôt international: PCT/EP2012/075829
(87) Numéro de publication internationale: WO 2013/087936

(56) Documents cités:
- WO-A1-2008/108549
- FR-A1- 2 937 973
- LT-B- 4 369
- US-A- 5 905 091
- DATABASE EMBASE [Online] ELSEVIER SCIENCE PUBLISHERS, AMSTERDAM, NL; 1979, HERNANDEZ-PEREZ E: "Vitiligo treated with ACTH", XP002677996, Database accession no. EMB-1980025160 & HERNANDEZ-PEREZ E: "Vitiligo treated with ACTH", INTERNATIONAL JOURNAL OF DERMATOLOGY 1979 US, vol. 18, no. 7, 1979, pages 578-579, ISSN: 0011-9059
- B. B. GOKHALE ET AL: "Corticotrophin and vitiligo (preliminary observations)", BRITISH JOURNAL OF DERMATOLOGY, vol. 95, no. 3, 1 septembre 1976 (1976-09-01), pages 329-329, XP055030280, ISSN: 0007-0963, DOI: 10.1111/j.1365-2133.1976.tb07022.x

## Description

L'invention concerne une association de composés pour le traitement et/ou la prévention des affections dermatologiques liées à une hypopigmentation.

La pigmentation de la peau humaine résulte de la synthèse de mélanine par les mélanocytes. Les mélanocytes contiennent des organelles appelées mélanosomes, siège de la biosynthèse de mélanine et, plus particulièrement, de deux types chimiquement distincts de mélanines, l'eumélanine, un pigment de couleur brun-noir et, la phaéomélanine, un pigment de couleur jaune-rouge. La synthèse de la mélanine ou mélanogénèse fait intervenir schématiquement les principales étapes suivantes :
Tyrosine -> Dopa -> DOPAquinone -> Dopachrome -> Mélanine.

A l'intérieur des mélanosomes, la tyrosinase est l'enzyme clé de la mélanogenèse. Elle est synthétisée sous la forme d'un précurseur inactif, qui est activé lorsque les mélanocytes sont stimulés par l'α-MSH via l'AMPc. La tyrosinase catalyse notamment les deux premières étapes avec l'hydroxylation de la tyrosine en 3,4-dihydroxyphenylalanine (DOPA) et l'oxydation de DOPA (intermédiaire non libre de la réaction catalytique) en DOPAquinone.

Tous les individus, sans distinction de phototype, ont approximativement le même nombre de mélanocytes pour une zone cutanée donnée non affectée. Les différences en terme de pigmentation ne sont pas dues au nombre de mélanocytes, mais aux propriétés de leurs mélanosomes. Chez l'homme, comme chez les autres mammifères, la couleur de la peau et des poils est principalement déterminée par le nombre, la taille, le type et le mode de répartition des mélanosomes. La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Ce sont les mélanosomes qui, après migration le long des dendrites, sont transférés des mélanocytes vers les kératinocytes et redistribués dans les kératinocytes. Les kératinocytes sont alors transportés à la surface de la peau au cours du processus de la différenciation de l'épiderme et la mélanine est ainsi répartie dans l'épiderme, assurant son brunissement et sa protection (Gilchrest BA, Park HY, Eller MS, Yaar M, Mechanisms of ultraviolet light-induced pigmentation. Photochem Photobiol 1996; 63: 1-10; Hearing VJ, Tsukamoto K, Enzymatic control of pigmentation in mammals. FASEB J 1991; 5: 2902-2909).

Bien que le taux de mélanine varie d'une population à une autre, la quantité de tyrosinase ne varie pas significativement et le taux d'ARN messagers de la tyrosinase est identique dans des peaux blanches ou noires.

Les variations dans la mélanogénèse sont donc notamment dues à des variations de l'activité des mélanocytes et principalement de la tyrosinase, au type de mélanine synthétisée, et à la capacité des kératinocytes à phagocyter les mélanosomes et à se répartir dans la peau.

De plus, on sait que la pigmentation naturelle peut être modulée par un grand nombre de facteurs intrinsèques ou extrinsèques. La quantité et la nature des mélanines contenues dans la peau humaine, ainsi que leur répartition, sont influencées par différents facteurs comme l'hérédité ou encore par une exposition à des agents physiques tels que les rayons X, UV, à une brûlure, au froid et autres agressions mécaniques ou encore à des agents chimiques dépigmentants tels que les dermocorticoïdes, l'acide azélaïque, le peroxyde de benzoyle, l'hydroquinone.

Parmi les différentes causes possibles d'hypopigmentation cutanée, on peut distinguer les hypopigmentations héréditaires diffuses ou circonscrites et les hypopigmentations acquises, non génétiquement déterminées, circonscrites.

Plus particulièrement, le vitiligo est une affection chronique de la peau caractérisée par l'apparition de taches d'un blanc mat à contour précis sur les pieds, les mains, le visage, les lèvres ou d'autres parties du corps. La dépigmentation peut être plus ou moins importante, et les zones dépigmentées, de tailles variables. À l'extrême, les poils ou les cheveux qui poussent à l'intérieur des régions dépigmentées sont également blancs. Le vitiligo touche de 1% à 2% de la population. Il apparaît généralement avant 20 ans (la moitié des personnes atteintes l'ont eu avant cet âge). Il n'est ni contagieux, ni souffrant, mais peut entraîner une certaine détresse psychologique en raison de son aspect inesthétique. Il existe deux types de vitiligo :
- le vitiligo segmentaire localisé de manière unilatérale sur une zone du visage, du haut du corps, des jambes ou des bras, qui en général n'évolue pas;
- le vitiligo généralisé, qui présente des taches souvent bilatérales plus ou moins symétriques sur des zones de friction ou de pression répétées et qui peut devenir plus important au fil des ans.

Les personnes atteintes de vitiligo ont une probabilité plus importante de développer d'autres maladies auto-immunes.

La dépigmentation observée dans le vitiligo résulte de la disparition des mélanocytes dans les zones lésionnelles. Plusieurs causes sont à l'origine de ce désordre: autoimmune, autocytotoxique, dysfonctionnement neurale, stress violent (Passeron et Ortonne, 2005).

De nombreuses solutions ont été proposées dans le domaine de la coloration naturelle, par stimulation des voies naturelles de pigmentation, par exemple en utilisant des actifs stimulant la mélanogénèse avec ou sans action des UV, comme l'α-MSH ou ses dérivés ou les prostaglandines. Des facteurs intrinsèques, qui régulent la pigmentation, peuvent également provenir non seulement des kératinocytes et des fibroblastes mais également des cellules endothéliales et des hormones véhiculées par l'apport sanguin, des cellules inflammatoires et du système nerveux. Dans les kératinocytes, Foxn1 et p53 sont des facteurs de transcription qui régulent la pigmentation cutanée via le FGFbeta et des dérivés de POMC tels que la α-MSH et l'ACTH, respectivement. D'autres activateurs kératinocytaires de la mélanogénèse sont le SCF/steel factor, l'HGF, le GM-CSF, le NGF, l'endorphine, l'endotheline-1 (ET-1), la prostaglandine (PG)E2/PGF2α et le LIF. Les cellules endothéliales sont des sources d'endothéline-1, de prostaglandines PGE2/PGF2, et d'oxyde nitrique qui activent la pigmentation cutanée. Les prostaglandines, les thromboxanes, et les leucotriènes augmentent l'activité tyrosinase et sont responsables de l'hyperpigmentation post-inflammatoire. Par contre, l'IL6 et le TNFalpha sont des inhibiteurs de la pigmentation cutanée. L'histamine, le NO, le GM-CSF, et l'α-MSH sont d'autres facteurs produits au cours de l'inflammation qui augmentent la mélanogénèse (M. Demarchez, Melanocyte and Pigmentation, Biology of Skin, 2011).

Ainsi, on connaît par exemple le document brevet US5905091, qui divulgue l'utilisation de prostaglandines pour stimuler la synthèse de mélanine dans les mélanocytes humains afin de favoriser le bronzage de la peau et augmenter la photo protection contre le rayonnement UV.

On connaît aussi les documents brevets WO2010/052255 et WO2010/052253, qui divulguent des composés modulateurs de MC1R utilisés pour traiter des désordres pigmentaires, à la fois hypopigmentaires et hyperpigmentaires, inflammatoires et immunitaires.

Toutefois, comme c'est le cas par exemple pour le vitiligo, l'hypopigmentation voire la dépigmentation résulte de la disparition des mélanocytes dans les zones lésionnelles. De tels traitements avec des prostaglandines ou des composés modulateurs de MC1R peuvent ainsi s'avérer peu efficaces voire inefficaces.

Par ailleurs, l'irradiation UV est également bien connue pour augmenter la plupart des facteurs qui stimulent la mélanogénèse. Les UV induisent une réponse immédiate et une réponse plus tardive. L'action immédiate, quelques minutes, persiste plusieurs jours mais cette augmentation rapide de la pigmentation résulte seulement de l'oxydation de pigments préexistants et de la redistribution des mélanosomes sans augmentation de la mélanogenèse. La réponse tardive aux UV correspond à une augmentation de la mélanogénèse, qui résulte d'une augmentation de l'expression de MITF, un régulateur majeur de la transcription de la pigmentation et de ces cibles en aval incluant Pmel17, MART-1, la tyrosinase, Tyrp1, Tyrp2/Dct. De plus, les mélanocytes épidermiques et également les kératinocytes répondent à une exposition aux UV en augmentant leurs productions en α-MSH et ACTH, qui, à leur tour, induisent une augmentation de l'expression de MC1R à la surface des mélanocytes et stimulent ainsi la mélanogénèse (M. Demarchez, Melanocyte and Pigmentation, Biology of Skin, 2011).

Toutefois, toute exposition excessive aux UV peut entraîner des lésions structurelles de la peau chez l'homme. A court terme, ces lésions provoquent des brûlures, une fragilisation du tissu cutané et des cicatrices et, à long terme, un vieillissement cutané photo-induit. Ce photovieillissement, provoqué par la dégradation du collagène présent dans la peau sous l'influence des UV, se manifeste par l'apparition de rides et la perte de l'élasticité cutanée.

De plus, l'exposition aux UV, qu'elle soit naturelle ou artificielle sous des lampes à UV par exemple, est un facteur de risque connu de cancer cutané.

Considérant ce qui précède, un problème que se propose de résoudre l'invention est de réaliser un traitement amélioré des affections dermatologiques liées à une hypopigmentation en augmentant la mélanogénèse.

Dans le cadre de l'invention, l'augmentation de la mélanogénèse se caractérise par une réponse immédiate et une réponse plus tardive par stimulation des voies naturelles de pigmentation et augmentation de l'activité de ces voies. De plus, l'objet de l'invention limite tout risque de lésions structurelles de la peau et de cancer cutané chez l'homme.

Sans vouloir être liée par un mécanisme d'action particulier, la Demanderesse a ainsi montré de manière inattendue et surprenante qu'une association d'au moins un agoniste des récepteurs des prostaglandines et d'au moins un agoniste du récepteur MC1R stimule la mélanogénèse. Une telle association apparaît efficace pour le traitement et/ou la prévention des affections dermatologiques liées à une hypopigmentation. De plus, cette association limite les risques d'effets secondaires quelle que soit la durée d'application de ce produit de combinaison. En particulier, un tel produit permet de corriger les hypopigmentations au niveau de la peau en augmentant la mélanogénèse de par un effet potentialisé appelé également potentialisation ou synergie.

Est ainsi décrite une méthode de traitement des affections dermatologiques liées à une hypopigmentation, comprenant au moins un agoniste des récepteurs des prostaglandines en association ou en combinaison avec au moins un agoniste du récepteur MC1R.

L'invention a pour objet un produit de combinaison comprenant au moins un agoniste des récepteurs des prostaglandines et au moins un agoniste du récepteur MC1R, comme médicament pour une utilisation simultanée, séparée ou étalée dans le temps pour le traitement et/ou la prévention des affections dermatologiques liées à une hypopigmentation.

Selon un aspect de l'invention, l'agoniste des récepteurs des prostaglandines et l'agoniste du récepteur MC1R sont présents au sein d'une même composition.

Selon un autre aspect de l'invention, l'agoniste des récepteurs des prostaglandines et l'agoniste du récepteur MC1R sont présents séparément l'un de l'autre au sein de compositions distinctes.

Ainsi, un autre objet de l'invention concerne une administration (ou un régime d'administration) d'une composition comprenant l'agoniste des récepteurs des prostaglandines administrée en premier, puis une composition comprenant l'agoniste du récepteur MC1R administrée en second.

Selon un aspect de l'invention, l'agoniste des récepteurs des prostaglandines est présent à une concentration comprise entre environ 0,001 et 1% en poids, par rapport au poids total de la composition le comprenant. L'agoniste du récepteur MC1R est présent à une concentration comprise entre environ 0,001% et 10% en poids par rapport au poids total de la composition le comprenant.

Dans un aspect préféré de l'invention, le produit de combinaison comprend au moins un agoniste des récepteurs des prostaglandines à une concentration comprise entre environ 0,004% et 0,04% et au moins un agoniste du récepteur MC1R à une concentration comprise entre environ 5% et 10% en poids par rapport au poids total de la composition le comprenant, comme médicament pour une utilisation simultanée, séparée ou étalée dans le temps pour le traitement et/ou la prévention des affections dermatologiques liées à une hypopigmentation.

Dans un aspect préféré de l'invention, la ou les compositions sont administrées par voie topique.

Selon un aspect particulier de l'invention, l'agoniste des récepteurs des prostaglandines est un agoniste PGE ou PGF ; de préférence il est un agoniste PGF2α et préférentiellement choisi parmi le latanoprost, le bimatoprost, le travoprost, le fluprostenol et l'unoprostone ; préférentiellement le travoprost.

Selon un aspect particulier de l'invention, l'agoniste du récepteur MC1R est choisi parmi les hormones stimulantes du mélanocytes (MSH) α, β ou γ, l'hormone adrénocorticotropique (ACTH) 1-39, la β-lipotropine, la β-endorphine et des composés de formule générale (I)ou (II) tels que définis par la suite.

Un autre objet de l'invention concerne un agoniste du récepteur MC1R pour une utilisation dans le traitement et/ou la prévention des affections dermatologiques liées à une hypopigmentation, en association ou en combinaison avec au moins un agoniste des récepteurs des prostaglandines, comme médicament pour une utilisation simultanée, séparée ou étalée dans le temps.

Un autre objet de l'invention se rapporte à une composition comprenant au moins un agoniste du récepteur MC1R et au moins un agoniste des récepteurs des prostaglandines, dans un véhicule physiologiquement acceptable.

### Description détaillée

L'invention et les avantages qui en découlent seront mieux compris à la lecture de la description et des modes de réalisation non limitatifs qui suivent.

Dans l'ensemble de la présente description, à moins qu'il ne soit spécifié autrement, il est entendu que, lorsque des intervalles de concentrations sont donnés, ils incluent les bornes supérieures et inférieures dudit intervalle.

L'invention vise le traitement et/ou la prévention des affections dermatologiques liées à une hypopigmentation ou une dépigmentation cutanée chez un patient.

Le patient est défini comme un humain quelque soit son âge.

On entend par traitement selon une définition générale, une amélioration ou un soulagement d'une partie ou de la totalité des symptômes majeurs de la pathologie ou de l'affection dermatologique. En particulier, le traitement favorise la mélanogénèse et/ou la migration des mélanocytes et/ou la différentiation et/ou la prolifération des mélanoblastes.

On entend par prévention, la limitation ou l'empêchement d'apparition des symptômes majeurs de l'affection dermatologique.

L'affection dermatologique liée à une hypopigmentation visée par l'invention est en particulier une dépigmentation ou une hypopigmentation et préférentiellement choisie parmi le vitiligo, l'albinisme, les hypomélanoses, les dépigmentations par des agents physiques ou chimiques, les hypopigmentations post-inflammatoires, le phénomène de Sutton ou toutes autres lésions hypopigmentaires, encore plus préférentiellement le vitiligo.

Par hypopigmentation on entend une diminution de la coloration habituelle de la peau, des poils ou cheveux, voire une décoloration totale résultant en une dépigmentation caractérisée par l'absence de mélanocytes dans la zone affectée.

L'invention comprend l'association ou la combinaison d'au moins un agoniste des récepteurs des prostaglandines et au moins un agoniste du récepteur MC1R.

L'agoniste des récepteurs des prostaglandines selon l'invention est préférentiellement un agoniste PGE ou PGF, plus préférentiellement un agoniste PGE2 et PGF2α, et encore plus préférentiellement un agoniste PGF2α.

L'agoniste PGF2α selon l'invention est préférentiellement choisi parmi le latanoprost, le bimatoprost, le travoprost, le fluprostenol et l'unoprostone, encore plus préférentiellement le travoprost.

L'agoniste du récepteur MC1R selon l'invention est préférentiellement choisi parmi les hormones stimulantes du mélanocytes (MSH) α, β ou γ, l'hormone adrénocorticotropique (ACTH) 1-39, la β-lipotropine, la β-endorphine et des composés de formule générale (I)ou (II) suivante : 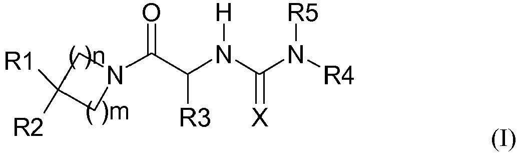 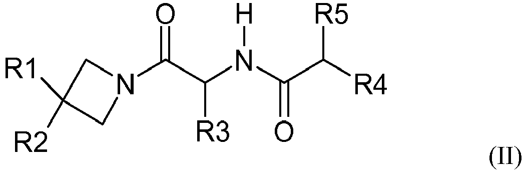 dans laquelle pour la formule (I):
R1 représente un atome d'hydrogène, un aryle, un aryle substitué, un alkyle, un cycloalkyle, un cycloalkylalkyle, ou un cycloalkylalkylalkyle ;
R2 représente un atome d'hydrogène, un hydroxy, un alkyle inférieur, un alkyle inférieur substitué, un alkyle supérieur, un alkyle supérieur substitué, un cycloalkyle, un cycloalkylalkyle, un alkoxy inférieur, un alkoxy inférieur substitué, un alkoxy supérieur, un alkoxy supérieur substitué, un cycloalkylalkoxy, un acyloxy, un acyle, un alkoxycarbonyl, un carboxamide, un acide carboxylique, un cyano, ou un amino disubstitué par un acyle et un aryle ou alkyle ;
R3 représente un aralkyle ou un aralkyle substitué ;
R4 représente un hétéroaralkyle ou un hétéroaralkyle substitué ;
R5 représente un atome d'hydrogène ou un alkyle ;
X représente un atome d'oxygène ou un atome de soufre ;
n, m peuvent être égaux à 1 ou 2 ;
ainsi que les sels et énantiomères correspondants.

Dans laquelle pour la formule générale(II)
R1 représente un aryle, un aryle substitué ou un cycloalkyle ;
R2 représente un atome d'hydrogène, un hydroxy, un alkyle inférieur, un alkyle inférieur substitué, un alkyle supérieur, un alkyle supérieur substitué, un cycloalkyle, un cycloalkylalkyle pouvant être substitué, un alkoxy inférieur, un alkoxy inférieur substitué, un alkoxy supérieur, un alkoxy supérieur substitué, un cycloalkylalkoxy, ou un acyloxy ;
R3 représente un aralkyle ou un aralkyle substitué;
R4 représente un hétéroaralkyle, un hétéroaralkyle substitué, un hétéroalkyle ou un hétéroalkyle substitué;
R5 représente un atome d'hydrogène, un hydroxy, un amino, un acylamino ou un sulfonamide ;
ainsi que les sels et énantiomères des composés de formule générale (I) correspondants.

Selon un des aspects préférés, dans la formule générale (II) :
R1 représente un cyclopropylméthyl ou un groupement 4-hydroxybutyl ;
R2 représente un atome d'hydrogène, un groupement méthyl.

Parmi les sels d'addition des composés de formule générale (I) ou (II) avec un acide pharmaceutiquement acceptable, on peut citer de préférence les sels avec un acide organique ou avec un acide inorganique.

Des acides inorganiques appropriés sont par exemple les acides halohydriques comme l'acide chlorhydrique ou l'acide bromhydrique, l'acide sulfurique, l'acide nitrique.

Des acides organiques appropriés sont par exemple l'acide picrique, l'acide méthane sulfonique, l'acide éthane sulfonique, l'acide para toluène sulfonique, l'acide oxalique, l'acide tartrique.

Les composés de formule générale (I)ou (II), peuvent également exister sous formes d'hydrates ou de solvates avec de l'eau ou avec un solvant.

Des solvants appropriés pour former des solvates ou des hydrates sont par exemple les alcools comme l'éthanol ou l'iso-propanol ou l'eau.

Selon la présente invention, un aryle désigne, en particulier, un phényle ou un naphtyle non substitué.

Selon la présente invention, un aryle substitué désigne, en particulier, un phényle ou un naphtyle substitué par un ou plusieurs groupes d'atome choisis parmi un alkyle, un alkoxy, un halogène, un hydroxy, un cyano, un trifluorométhyle et un nitro.

Selon la présente invention, un cycloalkyle désigne, en particulier, une chaîne hydrocarbonée saturée, cyclique, comprenant de 3 à 7 atomes de carbone.

Selon la présente invention, hydroxy désigne le groupement OH.

Selon la présente invention, amino désigne le groupement NH2.

Selon la présente invention, cyano désigne le groupement CN.

Selon la présente invention, un acide carboxylique désigne, en particulier, le groupement CO2H.

Selon la présente invention, un acyle désigne, en particulier, un formyl ou un carbonyl substitué par un alkyle, un cycloalkyle ou un cycloalkylalkyle.

Selon la présente invention, un alkyle désigne, en particulier, un alkyle inférieur ou un alkyle supérieur substitué ou non.

Selon la présente invention, un alkyle inférieur désigne, en particulier, une chaîne hydrocarbonée saturée, insaturée, linéaire ou ramifiée, comprenant de 1 à 4 atomes de carbone ou une chaîne hydrocarbonée insaturée comprenant de 2 à 4 atomes de carbone et notamment par exemple méthyle, éthyle, propyle, isopropyl, butyle.

Selon la présente invention, un alkyle inférieur substitué désigne, en particulier, une chaîne hydrocarbonée saturée, insaturée, linéaire ou ramifiée, comprenant de 1 à 4 atomes de carbone et substituée par un ou plusieurs atomes d'halogène ou par un hydroxy, ou une chaîne hydrocarbonée insaturée comprenant de 2 à 4 atomes de carbone et substituée par un ou plusieurs atomes d'halogène ou par un hydroxy.

Selon la présente invention, un alkyle supérieur désigne, en particulier, une chaîne hydrocarbonée saturée, insaturée, linéaire ou ramifiée, comprenant de 5 à 10 atomes de carbone.

Selon la présente invention, un alkyle supérieur substitué désigne, en particulier, une chaîne hydrocarbonée saturée, insaturée, linéaire ou ramifiée, comprenant de 5 à 10 atomes de carbone et substituée par un ou plusieurs atomes d'halogène ou par un hydroxy.

Selon la présente invention, un atome d'halogène désigne, en particulier, les atomes de chlore, fluor, iode et brome.

Selon la présente invention, un cycloalkylalkyle désigne, en particulier, un alkyle substitué par un cycloalkyle.

Selon la présente invention, un alkoxy inférieur désigne, en particulier, un atome d'oxygène substitué par un alkyle inférieur et notamment par exemple méthoxy, éthoxy, propoxy, isopropoxy, butoxy.

Selon la présente invention, un alkoxy inférieur substitué désigne, en particulier, un atome d'oxygène substitué par un alkyle inférieur substitué.

Selon la présente invention, un alkoxy supérieur désigne, en particulier, un atome d'oxygène substitué par un alkyle supérieur.

Selon la présente invention, un alkoxy supérieur substitué désigne, en particulier, un atome d'oxygène substitué par un alkyle supérieur substitué.

Selon la présente invention, un cycloalkylalkoxy désigne, en particulier, un atome d'oxygène substitué par un cycloalkylalkyle.

Selon la présente invention, un acyloxy désigne, en particulier, un atome d'oxygène substitué par un acyle.

Selon la présente invention, un alkoxycarbonyl désigne, en particulier, un carbonyl substitué par un alkoxy, cycloalkoxy ou un cycloalkylalkoxy.

Selon la présente invention, un carboxamide désigne, en particulier, un carbonyl substitué par un mono- ou un di-alkylamino.

Selon la présente invention, un aralkyle désigne, en particulier, un alkyle substitué par un aryle.

Selon la présente invention, un aralkyle substitué désigne, en particulier, un alkyle substitué par un aryle substitué.

Selon la présente invention, un hétérocycle désigne, en particulier, une chaîne hydrocarbonée cyclique ou bicyclique, saturée ou insaturée, comprenant un ou plusieurs hétéroatomes choisis parmi O, S et N.

Selon la présente invention, un hétérocycle substitué désigne, en particulier, une chaîne hydrocarbonée cyclique ou bicyclique, saturée ou insaturée, comprenant un ou plusieurs hétéroatomes choisis parmi O, S et N substitué par un ou plusieurs groupes alkyles.

Selon la présente invention, un hétéroaryle désigne, en particulier, un hétérocycle aromatique.

Selon la présente invention, un hétéroaryle substitué désigne, en particulier, un hétérocycle aromatique substitué par un ou plusieurs groupes alkyles.

Selon la présente invention, un hétéroaralkyle désigne, en particulier, un alkyle substitué par un hétéroaryle.

Selon la présente invention, un hétéroaralkyle substitué désigne, en particulier, un alkyle substitué par un hétéroaryle substitué.

L'agoniste du récepteur MC1R selon l'invention est plus préférentiellement choisi parmi les composés de formule générale (I) suivants :
1-[(S)-2-(4-Butyryl-4-phényl-pipéridin-1-yl)-1-(4-méthoxy-benzyl)-2-oxo-éthyl]-3-[2-(1H-imidazol-4-yl)-éthyl]-urée ;
1-[2-(1H-Imidazol-4-yl)-éthyl]-3-[1-(4-méthoxy-benzyl)-2-oxo-2-(4-oxo-1-phényl-1,3,8-triaza-spiro[4.5]déc-8-yl)-éthyl]-urée ;
1-[2-(4-Cyano-4-phényl-pipéridin-1-yl)-1-(4-méthoxy-benzyl)-2-oxo-éthyl]-3-[2-(1H-imidazol-4-yl)-éthyl]-urée ;
1-[2-(1H-Imidazol-4-yl)-éthyl]-3-[1-(4-méthoxy-benzyl)-2-oxo-2-(4-phényl-pipéridin-1-yl)-éthyl]-urée ;
1-[2-(1H-Imidazol-4-yl)-éthyl]-3-[1-(4-méthoxy-benzyl)-2-oxo-2-pipéridin-1-yl-éthyl]-urée ;
4-Cyclohexyl-1-[2-{3-[2-(1H-imidazol-4-yl)-éthyl]-uréido}-3-(4-méthoxy-phényl)-propionyl]-pipéridine-4-carboxylate d'éthyle ;
N-{1-[2-{3-[2-(1H-Imidazol-4-yl)-éthyl]-uréido}-3-(4-méthoxy-phényl)-propionyl]-pipéridin-4-yl}-N-phényl-propionamide ;
Butyrate de 1-[2-{3-[2-(1H-imidazol-4-yl)-éthyl]-uréido}-3-(4-méthoxy-phényl)-propionyl]-3-phényl-azétidin-3-yle ;
1-[2-{3-[2-(1H-Imidazol-4-yl)-éthyl]-uréido}-3-(4-méthoxy-phényl)-propionyl]-pipéridine-4-carboxylate d'éthyle ;
1-[2-(1H-Imidazol-4-yl)-éthyl]-3-{1-(4-méthoxy-benzyl)-2-[4-(2-méthoxy-phényl)-pipéridin-1-yl]-2-oxo-éthyl}-urée ;
1-[2-(3-Butoxy-3-phényl-azétidin-1-yl)-1-(4-méthoxy-benzyl)-2-oxo-éthyl]-3-[2-(1H-imidazol-4-yl)-éthyl]-urée ;
4-Cyclohexyl-1-[2-{3-[2-(1H-imidazol-4-yl)-éthyl]-uréido}-3-(4-méthoxy-phényl)-propionyl]-pipéridine-4-carboxylamide de méthyle ;
1-[2-(3-Cyclohexanecarbonyl-azétidin-1-yl)-1-(4-méthoxy-benzyl)-2-oxo-éthyl]-3-[2-(1H-imidazol-4-yl)-éthyl]-urée ;
4-Cyclohexyl-1-[2-{3-éthyl-3-[2-(1H-imidazol-4-yl)-éthyl]-uréido}-3-(4-méthoxy-phényl)-propionyl]-pipéridine-4-carboxylate d'éthyle ;
N-Cyclopropyl-N-{1-[2-{3-[2-(1H-imidazol-4-yl)-éthyl]-uréido}-3-(4-méthoxy-phényl)-propionyl]-pipéridin-4-yl}-propionamide ;
4-Cyclohexyl-1-(2-{3-[2-(1H-imidazol-4-yl)-éthyl]-uréido}-3-phényl-propionyl)-pipéridine-4-carboxylate d'éthyle ;
1-[2-(4-Butyryl-4-cyclohexyl-pipéridin-1-yl)-1-(4-méthoxy-benzyl)-2-oxo-éthyl]-3-[2-(3H-imidazol-4-yl)-éthyl]-urée ;
1-[2-(4-Butoxy-4-cyclohexyl-pipéridin-1-yl)-1-(4-méthoxy-benzyl)-2-oxo-éthyl]-3-[2-(1H-imidazol-4-yl)-éthyl]-urée ;
4-Cyclohexyl-1-(2-{3-[2-(1H-imidazol-4-yl)-éthyl]-uréido}-2-phényl-acétyl)-pipéridine-4-carboxylate d'éthyle ;
4-Cyclohexyl-1-[2-{3-[2-(1H-imidazol-4-yl)-éthyl]-uréido}-3-(4-méthoxy-phényl)-propionyl]-pipéridine-4-carboxylate de méthyle ;
1-[2-(4-Cyclohexyl-4-éthoxy-pipéridin-1-yl)-1-(4-méthoxy-benzyl)-2-oxo-éthyl]-3-[2-(1H-imidazol-4-yl)-éthyl]-urée ;
1-[2-(4-Acétyl-4-cyclohexyl-pipéridin-1-yl)-1-(4-méthoxy-benzyl)-2-oxo-éthyl]-3-[2-(1H-imidazo1-4-yl)-éthyl]-urée
4-Cyclohexyl-1-(2-{3-[2-(1H-imidazol-4-yl)-éthyl]-uréido}-2-phényl-acétyl)-pipéridine-4-carboxylate de méthyle ;
4-Ethyl-1-[2-{3-[2-(1H-imidazol-4-yl)-éthyl]-uréido}-3-(4-méthoxy-phényl)-propionyl]-pipéridine-4-carboxylate d'éthyle ;
1-[2-(4-Cyclohexyl-4-propoxy-pipéridin-1-yl)-1-(4-méthoxy-benzyl)-2-oxo-éthyl]-3-[2-(1H-imidazol-4-yl)-éthyl]-urée ;
Acide 4-cyclohexyl-1-[2-{3-[2-(1H-imidazo1-4-yl)-éthyl]-uréido}-3-(4-méthoxy-phényl)-propionyl]-pipéridine-4-carboxylique ;
1-[2-(1H-Imidazol-4-yl)-éthyl]-3-{1-(4-méthoxy-benzyl)-2-[3-(2-méthyl-cyclohexyl)-3-propoxy-azétidin-1-yl]-2-oxo-éthyl}-urée ;
4-Cyclohexyl-1-[2-{3-[2-(1H-imidazol-4-yl)-éthyl]-uréido}-3-(4-méthoxy-phényl)-propionyl]-pipéridine-4-carboxylate de propyle ;
1-[2-(1H-Imidazol-4-yl)-éthyl]-3-[1-(4-méthoxy-benzyl)-2-oxo-2-(3-pentyl-3-phényl-azétidin-1-yl)-éthyl]-urée ;
1-((R)-3-(4-Chloro-phényl)-2-{3-[2-(1H-imidazol-4-yl)-éthyl]-uréido}-propionyl)-4-cyclohexyl-pipéridine-4-carboxylate d'éthyle ;
1-((S)-3-(4-Chloro-phényl)-2-{3-[2-(1H-imidazol-4-yl)-éthyl]-uréido}-propionyl)-4-cyclohexyl-pipéridine-4-carboxylate d'éthyle ;
1-[2-(4-Cyclohexyl-4-propionyl-pipéridin-1-yl)-1-(4-méthoxy-benzyl)-2-oxo-éthyl]-3-[2-(1H-imidazol-4-yl)-éthyl]-urée ;
1-[2-(4-Cyclohexyl-4-propionyl-pipéridin-1-yl)-1-(4-méthoxy-benzyl)-2-oxo-éthyl]-3-(1H-imidazol-4-ylméthyl)-urée ;
4-Cyclohexyl-1-[(R)-2-{3-[2-(1H-imidazol-4-yl)-éthyl]-uréido}-3-(4-méthoxy-phényl)-propionyl]-pipéridine-4-carboxylate d'éthyle ;
4-Cyclopropylméthyl-1-[2-{3-[2-(1H-imidazol-4-yl)-éthyl]-uréido}-3-(4-méthoxy-phényl)-propionyl]-pipéridine-4-carboxylate d'éthyle ;
4-Cyclohexyl-1-(2-{3-[2-(1H-imidazol-4-yl)-éthyl]-uréido}-3-phényl-propionyl)-pipéridine-4-carboxylate de propyle ;
4-Cyclopentyl-1-(2-{3-[2-(1H-imidazol-4-yl)-éthyl]-uréido}-3-phényl-propionyl)-pipéridine-4-carboxylate d'éthyle ;
4-Cyclopentyl-1-[2-{3-[2-(1H-imidazol-4-yl)-éthyl]-uréido}-3-(4-méthoxy-phényl)-propionyl]-pipéridine-4-carboxylate d'éthyle ;
4-Cyclohexyl-1-[(S)-2-{3-[2-(1H-imidazol-4-yl)-éthyl]-uréido}-3-(4-méthoxy-phényl)-propionyl]-pipéridine-4-carboxylate d'éthyle ;
1-[(R)-2-(4-Butyryl-4-cyclohexyl-pipéridin-1-yl)-1-(4-méthoxy-benzyl)-2-oxo-éthyl]-3-[2-(1H-imidazol-4-yl)-éthyl]-urée ;
1-[(R)-2-(4-Butyryl-4-cyclohexyl-pipéridin-1-yl)-1-(4-fluoro-benzyl)-2-oxo-éthyl]-3-[2-(1H-imidazol-4-yl)-éthyl]-urée ;
1-[(R)-1-Benzyl-2-(4-butyryl-4-cyclohexyl-pipéridin-1-yl)-2-oxo-éthyl]-3-[2-(1H-imidazol-4-yl)-éthyl]-urée ;
1-[(R)-2-(4-Butyryl-4-cyclohexyl-pipéridin-1-yl)-1-(4-methoxy-benzyl)-2-oxo-éthyl]-3-[2-(3-méthyl-3H-imidazol-4-yl)-éthyl]-urée ;
1-[(R)-2-(4-Butyryl-4-cyclohexyl-pipéridin-1-yl)-1-(4-chloro-benzyl)-2-oxo-éthyl]-3-[2-(1H-imidazol-4-yl)-éthyl]-urée ;
4-Cyclohexyl-1-((R)-3-(3,4-dichloro-phényl)-2-{3-[3-(1H-imidazol-4-yl)-propyl]-uréido}-propionyl)-pipéridine-4-carboxylate d'éthyle ;
4-Cyclohexyl-1-((R)-3-(4-méthoxy-phényl)-2-{3-[2-(3-méthyl-3H-imidazol-4-yl)-éthyl]-uréido}-propionyl)-pipéridine-4-carboxylate d'éthyle ;
4-Cyclohexyl-1-[(R)-2-{3-[2-(1H-imidazol-4-yl)-éthyl]-thiouréido}-3-(4-méthoxy-phényl)-propionyl]-pipéridine-4-carboxylate d'éthyle ;
1-[(R)-2-(4-Butyryl-4-cyclohexyl-pipéridin-1-yl)-1-(4-méthoxy-benzyl)-2-oxo-éthyl]-3-[2-(1H-imidazol-4-yl)-éthyl]-thiourée ;
1-[(R)-2-(4-Cyclohexyl-4-propoxy-pipéridin-1-yl)-1-(4-méthoxy-benzyl)-2-oxo-éthyl]-3-[2-(1H-imidazol-4-yl)-éthyl]-thiourée ;
1-[(R)-1-Benzyl-2-(4-cyclohexyl-4-propoxy-pipéridin-1-yl)-2-oxo-éthyl]-3-[2-(1H-imidazol-4-yl)-éthyl]-thiourée ;
1-[(R)-1-Benzyl-2-(4-cyclohexyl-4-propoxy-pipéridin-1-yl)-2-oxo-éthyl]-3-[2-(1H-imidazol-4-yl)-éthyl]-urée ;
4-Cyclohexyl-1-((R)-3-(4-méthoxy-phényl)-2-{3-[2-(3-méthyl-3H-imidazol-4-yl)-éthyl]-thiouréido}-propionyl)-pipéridine-4-carboxylate d'éthyle ;
4-Cyclohexyl-1-((R)-2-{3-[2-(3-méthyl-3H-imidazol-4-yl)-éthyl]-uréido}-3-phényl-propionyl)-pipéridine-4-carboxylate d'éthyle ;
1-[(R)-2-(4-Cyclohexyl-4-propoxy-pipéridin-1-yl)-1-(4-méthoxy-benzyl)-2-oxo-éthyl]-3-[2-(3-méthyl-3H-imidazol-4-yl)-éthyl]-urée ;
1-((R)-3-(4-Chloro-phényl)-2-{3-[2-(3-méthyl-3H-imidazol-4-yl)-éthyl]-uréido}-propionyl)-4-cyclohexyl-pipéridine-4-carboxylate d'éthyle ;
4-Cyclohexyl-1-((R)-3-(4-fluoro-phényl)-2-{3-[2-(3-méthyl-3H-imidazol-4-yl)-éthyl]-uréido}-propionyl)-pipéridine-4-carboxylate d'éthyle ;
4-Cyclohexyl-1-((R)-3-(4-fluoro-phényl)-2-{3-[2-(1H-imidazol-4-yl)-éthyl]-uréido}-propionyl)-pipéridine-4-carboxylate d'éthyle ;
4-Cyclohexyl-1-((R)-3-(4-fluoro-phényl)-2-{3-[2-(1H-imidazol-4-yl)-éthyl]-thiouréido}-propionyl)-pipéridine-4-carboxylate d'éthyle ;
1-((R)-3-(4-Chloro-phényl)-2-{3-[2-(1H-imidazol-4-yl)-éthyl]-thiouréido}-propionyl)-4-cyclohexyl-pipéridine-4-carboxylate d'éthyle ;
1-((R)-3-(4-Chloro-phényl)-2-{3-[2-(3-méthyl-3H-imidazol-4-yl)-éthyl]-thiouréido}-propionyl)-4-cyclohexyl-pipéridine-4-carboxylate d'éthyle ;
1-[(R)-2-(4-Cyclohexyl-4-propoxy-pipéridin-1-yl)-1-(4-fluoro-benzyl)-2-oxo-éthyl]-3-[2-(1H-imidazol-4-yl)-éthyl]-urée ;
1-[(R)-1-(4-Chloro-benzyl)-2-(4-cyclohexyl-4-propoxy-pipéridin-1-yl)-2-oxo-éthyl]-3-[2-(1H-imidazol-4-yl)-éthyl]-urée ; ainsi que leurs sels et énantiomères respectifs.

L'agoniste MC1R selon l'invention est encore plus préférentiellement le 4-Cyclohexyl-1-[(R)-2-{3-[2-(1H-imidazol-4-yl)-éthyl]-uréido}-3-(4-méthoxy-phényl)-propionyl]-pipéridine-4-carboxylate d'éthyle.

L'agoniste du récepteur MC1R selon l'invention est plus préférentiellement choisi parmi les composés de formule générale (II) suivants:
1-[(S)-2-[(S)-2-benzoylamino-3-(1H-imidazol-4-yl)-propionylamino]-3-(4-méthoxy-phényl)-propionyl]-3-phényl-azétidin-3-yle Butyric acid ester
   - N-[(S)-1-[(S)-2-(3-Butoxy-3-o-tolyl-azétidin-1-yl)-1-(4-méthoxy-benzyl)-2-oxo-éthylcarbamoyl]-2-(1H-imidazol-4-yl)-éthyl]-benzamide
   - N-[(S)-1-[(S)-2-(3-Butoxy-3-phényl- azétidin-1-yl)-1-(4-méthoxy-benzyl)-2-oxo-éthylcarbamoyl]-2-(1H-imidazol-4-yl)-éthyl]-benzamide
   - N-[(S)-1-[(S)-2-(3-Hydroxy-3-o-tolyl-azétidin-1-yl)-1-(4-méthoxy-benzyl)-2-oxoéthylcarbamoyl]-2-(1H-imidazol-4-yl)-éthyl]-benzamide
   - Acétate de 1-[(S)-2-[(S)-2-benzoyl amino-3-(1H-imidazol-4-yl)-propionylamino]-3-(4-méthoxy-phényl)-propionyl]-3-o-tolyl-azétidin-3-yle
   - Acide butyrique de 1-[(S)-2-[(S)-2-benzoylamino-3-(1H-imidazol-4-yl)-propionylamino]-3-(4-méthoxy-phényl)-propionyl]-3-(4-fluoro-phényl)-azétidin- 3-yle ester
   - N-[(S)-1-[(S)-2-(3-Cyclohexyl-3-hydroxy-azétidin-1-yl)-1-(4-méthoxy-benzyl)-2-oxo-éthylcarbamoyl]-2-(1H-imidazol-4-yl)-éthyl]-benzamide
   - N-[(S)-1-[(S)-2-[3-Butoxy-3-(4-fluoro-phényl)-azétidin-1-yl]-1-(4-méthoxy-benzyl)-2-oxo-éthylcarbamoyl]-2-(1H-imidazol-4-yl)-éthyl]-benzamide
   - N-[(S)-1-[(S)-2-[3-Butoxy-3-(3-fluoro-phényl)-azétidin-1-yl]-1-(4-méthoxy-benzyl)-2-oxo-éthylcarbamoyl]-2-(1H-imidazol-4-yl)-éthyl]-benzamide
   - N-[(S)-2-(3-Cyclohexyl-3-hydroxy-azétidin-1-yl)-1-(4-méthoxy-benzyl)-2-oxo-éthyl]-3-(1H-imidazol-4-yl)-propionamide
   - N-[(S)-2-(3-Hydroxy-3-o-tolyl-azétidin-1-yl)-1-(4-méthoxy-benzyl)-2-oxo-éthyl]-3-(1H-imidazol-4-yl)-propionamide
   - N-[(R)-1-(3,4-Dichloro-benzyl)-2-(3-hydroxy-3-phényl-azétidin-1-yl)-2-oxo-éthyl]-3-(1H-imidazol-4-yl)-propionamide
   - N-[(S)-2-(3-Ethoxy-3-o-tolyl-azétidin-1-yl)-1-(4-méthoxy-benzyl)-2-oxo-éthyl]-3-(1H-imidazol-4-yl)-propionamide
   - N-[(S)-2-(3-Cyclopropylméthoxy-3-o- tolyl-azétidin-1-yl)-1-(4-méthoxy-benzyl)-2-oxo-éthyl]-3-(1H-imidazol-4-yl)-propionamide
   - N-[(R)-2-(3-Butoxy-3-o-tolyl-azétidin-1-yl)-1-(4-méthoxy-benzyl)-2-oxo-éthyl]-3-(1H-imidazol-4-yl)-propionamide
   - N-[(S)-2-(3-Butoxy-3-o-tolyl-azétidin-1-yl)-1-(4-méthoxy-benzyl)-2-oxo-éthyl]-3-(1H-imidazo1-4-yl)-propionamide
   - N-[(R)-2-(3-Ethoxy-3-o-tolyl-azétidin-1-yl)-1-(4-méthoxy-benzyl)-2-oxo-éthyl]-3-(1H-imidazol-4-yl)-propionamide
   - N-[(R)-2-(3-Butoxy-3-phényl-azétidin-1-yl)-1-cyclohexylméthyl-2-oxo-éthyl]-3-(1H-imidazol-4-yl)-propionamide
   - N-[(R)-2-(3-Butoxy-3-phényl-azétidin-1-yl)-1-(2,4-dichloro-benzyl)-2-oxo-éthyl]-3-(1H-imidazol-4-yl)-propionamide
   - N-[(R)-2-(3-Cyclopropylméthoxy-3-o-tolyl-azétidin-1-yl)-1-(4-méthoxy-benzyl)-2-oxo-éthyl]-3-(1H-imidazol-4-yl)-propionamide
   - N-[(S)-1-(4-Méthoxy-benzyl)-2-oxo-2-(3-propoxy-3-o-tolyl-azétidin-1-yl)-éthyl]-3-(1H-imidazol-4-yl)-propionamide
   - N-[(R)-1-(4-Méthoxy-benzyl)-2-oxo-2-(3-propoxy-3-o-tolyl-azétidin-1-yl)-éthyl]-3-(1H-imidazol-4-yl)-propionamide
   - N-[(R)-2-[3-Butoxy-3-(4-fluoro-phényl)-azétidin-1-yl]-1-(4-méthoxy-benzyl)-2-oxo-éthyl]-3-(1H-imidazol-4-yl)-propionamide
   - N-[(R)-1-(4-Méthoxy-benzyl)-2-oxo-2-(3-pentyloxy-3-o-tolyl-azétidin-1-yl)-éthyl]-3-(1H-imidazol-4-yl)-propionamide
   - N-[(R)-2-(3-Hexyloxy-3-o-tolyl-azétidin-1-yl)-1-(4-méthoxy-benzyl)-2-oxo-éthyl]-3-(1H-imidazol-4-yl)-propionamide
   - N-[(R)-1-(4-Méthoxy-benzyl)-2-oxo-2-(3-pentyl-3-phenyl-azétidin-1-yl)-éthyl]-3-(1H-imidazol-4-yl)-propionamide
   - N-[(R)-2-(3-Butyl-3-phényl-azétidin-1-yl)-1-(4-méthoxy-benzyl)-2-oxo-éthyl]-3-(1H-imidazol-4-yl)-propionamide
   - N-[(R)-2-(3-Cyclopropylméthoxy-3-phényl-azétidin-1-yl)-1-(4-méthoxy-benzyl)-2-oxo-éthyl]-3-(1H-imidazol-4-yl)-propionamide
   - N-[(R)-2-(3-Hydroxy-3-o-tolyl-azétidin-1-yl)-1-(4-méthoxy-benzyl)-2-oxo-éthyl]-3-(1H-imidazol-4-yl)-propionamide
   - N-[(R)-2-[3-Butoxy-3-(4-fluoro-phényl)-azétidin-1-yl]-1-(3-fluoro-benzyl)-2-oxo-éthyl]-3-(1H-imidazol-4-yl)-propionamide
   - N-[(R)-2-[3-Butoxy-3-(4-fluoro-phényl)-azétidin-1-yl]-1-(4-fluoro-benzyl)-2-oxo-éthyl]-3-(1H-imidazol-4-yl)-propionamide
   - N-[(R)-2-(3-Butoxy-3-phényl-azétidin-1-yl)-1-(4-fluoro-benzyl)-2-oxo-éthyl]-3-(1H-imidazol-4-yl)-propionamide
   - N-{(R)-1-Benzyl-2-[3-butoxy-3-(4-fluoro-phényl)-azétidin-1-yl]-2-oxo-éthyl}-3-(4H-imidazol-2-yl)-propionamide
   - N-[(R)-1-Benzyl-2-(3-butoxy-3-phényl-azétidin-1-yl)-2-oxo-éthyl]-3-(1H-imidazol-4-yl)-propionamide
   - N-[(R)-2-(3-Butoxy-3-phényl-azétidin-1-yl)-1-(4-méthoxy-benzyl)-2-oxo-éthyl]-3-(1H-imidazol-4-yl)-propionamide
   - N-[(R)-1-(4-Méthoxy-benzyl)-2-oxo-2-(3-pentyl-3-o-tolyl-azétidin-1-yl)-éthyl]-3-(1H-imidazol-4-yl)-propionamide
   - N-[(R)-2-[3-(4-Fluoro-phényl)-3-pentyl-azétidin-1-yl]-1-(4-méthoxy-benzyl)-2-oxo-éthyl]-3-(1H-imidazol-4-yl)-propionamide
   - N-[(R)-2-(3-Butoxy-3-o-tolyl-azétidin-1-yl)-1-(4-méthoxy-benzyl)-2-oxo-éthyl]-3-(1H-[1,2,3]triazol-4-yl)-propionamide
   - N-[(R)-2-(3-Butoxy-3-o-tolyl-azétidin-1-yl)-1-(4-méthoxy-benzyl)-2-oxo-éthyl]-3-(5-méthyl-3H-[1,2,4]triazol-3-yl)-propionamide
   - N-[(R)-2-(3-Butoxy-3-o-tolyl-azétidin-1-yl)-1-(4-méthoxy-benzyl)-2-oxo-éthyl]-3-(5-méthyl-3H-imidazol-4-yl)-propionamide
   - N-{(R)-1-(4-Méthoxy-benzyl)-2-[3-(2-méthoxy-phényl)-3-pentyl-azétidin-1-yl]-2-oxo-éthyl}-3-(1H-imidazol-4-yl)-propionamide
   - N-[(R)-2-[3-(2-Fluoro-phényl)-3-pentyl-azétidin-1-yl]-1-(4-méthoxy-benzyl)-2-oxo-éthyl]-3-(1H-imidazol-4-yl)-propionamide
   - N-[(R)-2-[3-(2-Chloro-phényl)-3-pentyl-azétidin-1-yl]-1-(4-méthoxy-benzyl)-2-oxo-éthyl]-3-(1H-imidazol-4-yl)-propionamide
   - N-[(R)-1-(4-Chloro-benzyl)-2-oxo-2-(3-pentyl-3-phényl-azétidin-1-yl)-éthyl]-3-(1H-Imidazol-4-yl)-propionamide
   - N-[(R)-1-(4-Fluoro-benzyl)-2-oxo-2-(3-pentyl-3-phényl-azétidin-1-yl)-éthyl]-3-(1H-imidazol-4-yl)-propionamide
   - N-[(R)-1-Benzyl-2-oxo-2-(3-pentyl-3-phényl-azétidin-1-yl)-éthyl]-3-(1H-imidazol-4-yl)-propionamide
   - N-[(R)-2-(3-Butoxy-3-o-tolyl-azétidin-1-yl)-1-(4-méthoxy-benzyl)-2-oxo-éthyl]-3-(1H-imidazol-4-yl)-acrylamide
   - N-[(R)-2-[3-(2,4-Difluoro-phényl)-3-pentyl-azétidin-1-yl]-1-(4-méthoxy-benzyl)-2-oxo-éthyl]-3-(1H-imidazol-4-yl)-propionamide
   - N-[(R)-2-Oxo-2-(3-pentyl-3-phényl-azétidin-1-yl)-1-(3-trifluorométhyl-benzyl)-éthyl]- 3-(1H-imidazol-4-yl)-propionamide
   - N-[(R)-2-Oxo-2-(3-pentyl-3-phényl-azétidin-1-yl)-1-(4-trifluorométhyl-benzyl)-éthyl]- 3-(1H-imidazol-4-yl)-propionamide
   - N-[(R)-1-(3,4-Dichloro-benzyl)-2-oxo-2-(3-pentyl-3-phényl-azétidin-1-yl)-éthyl]-3-(1H-imidazol-4-yl)-propionamide
   - N-[(R)-1-(3,4-Difluoro-benzyl)-2-oxo-2-(3-pentyl-3-phényl-azétidin-1-yl)-éthyl]-3-(1H-imidazol-4-yl)-propionamide
   - N-[(R)-2-[3-(3,4-Dichloro-phényl)-3-pentyl-azétidin-1-yl]-1-(4-méthoxy-benzyl)-2-oxo-éthyl]-3-(1H-imidazol-4-yl)-propionamide
   - N-[(R)-2-[3-(3-Fluoro-phényl)-3-pentyl-azétidin-1-yl]-1-(4-méthoxy-benzyl)-2-oxo-éthyl]-3-(1H-imidazol-4-yl)-propionamide
   - N-[(R)-1-(3-Fluoro-benzyl)-2-oxo-2-(3-pentyl-3-phényl-azétidin-1-yl)-éthyl]-3-(1H-imidazol-4-yl)-propionamide
   - N-[(R)-1-(2-Fluoro-benzyl)-2-oxo-2-(3-pentyl-3-phényl-azétidin-1-yl)-éthyl]-3-(1H-imidazol-4-yl)-propionamide
   - N-[(R)-1-(2,4-Dichloro-benzyl)-2-oxo-2-(3-pentyl-3-phényl-azétidin-1-yl)-éthyl]-3-(1H-imidazol-4-yl)-propionamide
   - N-[(R)-2-[3-(4-Chloro-phényl)-3-pentyl-azétidin-1-yl]-1-(4-méthoxy-benzyl)-2-oxo-éthyl]-3-(1H-imidazol-4-yl)-propionamide
   - N-[(R)-2-[3-(2,5-Difluoro-phényl)-3-pentyl-azétidin-1-yl]-1-(4-méthoxy-benzyl)-2-oxo-éthyl]-3-(1H-imidazol-4-yl)-propionamide
   - N-[(R)-2-[3-(2,6-Difluoro-phényl)-3-pentyl-azétidin-1-yl]-1-(4-méthoxy-benzyl)-2-oxo-éthyl]-3-(1H-imidazol-4-yl)-propionamide
   - N-[2-(3-Butoxy-3-o-tolyl-azétidin-1-yl)-1-(4-méthoxy-benzyl)-2-oxo-éthyl]-3-(1H-imidazol-4-yl)-hexyramide
   - N-[2-(3-Butoxy-3-o-tolyl-azétidin-1-yl)-1-(4-méthoxy-benzyl)-2-oxo-éthyl]-3-(1H-imidazol-4-yl)-pentyramide
   - N-[(R)-1-(4-Méthoxy-benzyl)-2-oxo-2-(3-pentyl-3-phényl-azétidin-1-yl)-éthyl]-3-(3-méthyl-3H-imidazol-4-yl)-propionamide
   - N-[2-(3-Butoxy-3-o-tolyl-azétidin-1-yl)-1-(2,4-dichloro-benzyl)-2-oxo-éthyl]-3-(1H-imidazol-4-yl)-hexyramide
   - N-[(R)-2-(3-Cyclohexyl-3-pentyl-azétidin-1-yl)-1-(4-méthoxy-benzyl)-2-oxo-éthyl]-3-(1H-imidazol-4-yl)-propionamide
   - N-[(R)-2-(3-Butoxy-3-o-tolyl-azétidin-1-yl)-1-(4-méthoxy-benzyl)-2-oxo-éthyl]-3-(3-méthyl-3H-imidazol-4-yl)-propionamide
   - 3-(1H-Imidazol-4-yl)-N-[(R)-1-(4-méthoxy-benzyl)-2-oxo-2-(3-phényl-azétidin-1-yl)-éthyl]-propionamide
   - N-[(R)-2-[3-(4-Fluoro-phényl)-azétidin-1-yl]-1-(4-méthoxy-benzyl)-2-oxo-éthyl]-3-(1H-imidazol-4-yl)-propionamide
   - 3-(1H-Imidazol-4-yl)-N-{(R)-1-(4-méthoxy-benzyl)-2-[3-(2-méthoxy-phényl)-azétidin-1-yl]-2-oxo-éthyl}-propionamide
   - N-[(R)-2-[3-(2-Fluoro-phényl)-azétidin-1-yl]-1-(4-méthoxy-benzyl)-2-oxo-éthyl]-3-(1H-imidazol-4-yl)-propionamide
   - 3-(1H-Imidazol-4-yl)-N-{(R)-1-(4-méthoxy-benzyl)-2-oxo-2-[3-phényl-3-(4,4,4-trifluoro-butyl)-azétidin-1-yl]-éthyl}-propionamide
   - N-[(R)-2-[3-(5-Fluoro-pentyl)-3-phényl-azétidin-1-yl]-1-(4-méthoxy-benzyl)-2-oxo-éthyl]-3-(1H-imidazol-4-yl)-propionamide
   - N-[(R)-2-(3-Cyclopropyl-3-phényl-azétidin-1-yl)-1-(4-méthoxy-benzyl)-2-oxo-éthyl]-3-(1H-imidazol-4-yl)-propionamide
   - N-[(R)-2-(3-Cyclopropylméthyl-3-phényl-azétidin-1-yl)-1-(4-méthoxy-benzyl)-2-oxo-éthyl]-3-(1H-imidazol-4-yl)-propionamide
   - (S)-2-Hydroxy-3-(1H-imidazol-4-yl)-N-[1-(4-méthoxy-benzyl)-2-oxo-2-(3-propoxy-3-o-tolyl-azétidin-1-yl)-éthyl]-propionamide
   - (S)-2-Amino-3-(1H-imidazol-4-yl)-N-[1-(4-méthoxy-benzyl)-2-oxo-2-(3-propoxy-3-o-tolyl-azétidin-1-yl)-éthyl]-propionamide
   - N-[2-(3-Butoxy-3-o-tolyl-azétidin-1-yl)-1-(4-méthoxy-benzyl)-2-oxo-éthyl]-3-(1H-imidazol-4-yl)-butyramide
   - (S)-N-[2-(3-Butoxy-3-phényl-azétidin-1-yl)-1-(4-méthoxy-benzyl)-2-oxo-éthyl]-3-(1H-imidazol-4-yl)-2-méthanesulfonylamino-propionamide
   - N-[(R)-1-(4-Méthoxy-benzyl)-2-oxo-2-(3-pentyl-3-phényl-azétidin-1-yl)-éthyl]-3-(1-méthyl-1H-imidazol-4-yl)-propionamide
   - N-[(R)-2-[3-(4-Hydroxy-butoxy)-3-o-tolyl-azetidin-1-yl]-1-(4-methoxy-benzyl)-2-oxo-ethyl]-3-(-1H-imidazol-4-yl)-propionamide.
   - N-[(R)-2-(3-Cyclopropylmethoxy-3-o-tolyl-azetidin-1-yl)-1-(4-methoxy-benzyl)-2-oxo-ethyl]-3-(5-methyl-1H-imidazol-4-yl)-propionamide

Ainsi que leurs sels et leurs énantiomères correspondants.

Selon un des aspects préférés de l'invention, les composés de formule (II) suivants sont également choisis :
N-[(R)-2-[3-(4-Hydroxy-butoxy)-3-o-tolyl-azetidin-1-yl]-1-(4-methoxy-benzyl)-2-oxo-ethyl]-3-(-1H-imidazol-4-yl)-propionamide.
N-[(R)-2-(3-Cyclopropylmethoxy-3-o-tolyl-azetidin-1-yl)-1-(4-methoxy-benzyl)-2-oxo-ethyl]-3-(5-methyl-1H-imidazol-4-yl)-propionamide
ainsi que les sels et énantiomères correspondants.

Dans le cadre de la présente invention, un produit de combinaison comprenant un agoniste des récepteurs des prostaglandines et un agoniste du récepteur MC1R, signifie que lesdits composés associés peuvent être soit présents au sein d'une même composition (définie comme une combinaisons ou combinaison fixe), soit présents séparément l'un de l'autre au sein de compositions distinctes. En d'autres termes, ces composés sont destinés à être administrés dans le cadre d'un même traitement, c'est-à-dire sur une période commune de traitement, soit en même temps, en étant compris ou pas au sein d'une seule et même composition, soit en des instants différents. De plus, ils peuvent être administrés par des modes d'administration identiques ou différents et/ou être compris dans des compositions identiques ou différentes.

Une utilisation simultanée selon l'invention est une administration des composés du produit selon l'invention compris dans une seule et même composition pharmaceutique.

Une utilisation séparée selon l'invention est une administration, en même temps, des deux composés du produit selon l'invention compris chacun dans une composition pharmaceutique distincte, dans une formulation identique ou différente.

Une utilisation étalée dans le temps selon l'invention est une administration successive, des deux composés du produit selon l'invention chacun dans une composition pharmaceutique distincte, dans une formulation identique ou différente.

De façon générale, le produit selon l'invention augmente l'efficacité de la prévention et du traitement d'un désordre dermatologique liée à une hypopigmentation en augmentant la mélanogénèse. Dans le cadre de l'invention, l'augmentation de la mélanogénèse se caractérise par une réponse immédiate et une réponse plus tardive par stimulation des voies naturelles de pigmentation et augmentation de l'activité de ces voies.

Selon un mode de réalisation de l'invention, l'administration d'un produit selon l'invention comprenant au moins l'agoniste des récepteurs des prostaglandines et au moins l'agoniste du récepteur MC1R au sein d'une même composition, permet une action immédiate, simultanée et complémentaire sur les mélanocytes de la zone hypopigmentée à traiter et/ou proche de cette zone, et dans les cas de dépigmentation, sur les mélanocytes de la zone avoisinant la zone dépigmentée à traiter, de sorte à stimuler la mélanogénèse.

La demanderesse a découvert de manière inattendue et comme illustré dans l'exemple, que l'agoniste des récepteurs des prostaglandines qui agit immédiatement (action immédiate telle que définie précédemment) en augmentant la synthèse de phaéomélanine notamment, permet également de potentialiser l'action de l'agoniste du récepteur MC1R. En effet, dans le vitiligo, on observe une dépigmentation qui résulte de la disparition des mélanocytes dans les zones lésionnelles dépigmentées. La demanderesse a montré que le composé agoniste des récepteurs des prostaglandines, permettait la stimulation des cellules souches présentes au niveau basal et active leur différentiation en mélanocytes dans les zones lésionnelles qui en étaient dépourvues. Cette différentiation permet alors l'induction de l'expression du récepteur MC1R au niveau des mélanocytes. Ainsi, en révélant l'expression de récepteurs MC1R au niveau des cellules souches, l'administration en premier de l'agoniste des récepteurs des prostaglandines va venir potentialiser l'effet de l'agoniste du récepteur MC1R lors de son administration dans un second temps. Le composé agoniste du récepteur MC1R agit alors sur les récepteurs MC1R des mélanocytes nouvellement formés en entraînant une stimulation importante de l'activité tyrosinase et de la synthèse des eumélanines. De plus, l'administration d'un agoniste MC1R, augmente avantageusement les niveaux d'ARNm MC1R dans les mélanocytes induisant une augmentation de l'expression de MC1R à la surface des mélanocytes de manière à offrir une réponse plus importante à l'hypopigmentation voire la dépigmentation

Selon un mode de réalisation avantageux de l'invention, lors de l'administration d'un produit selon l'invention présentant l'agoniste des récepteurs des prostaglandines et l'agoniste du récepteur MC1R dans deux compositions distinctes, l'administration en même temps de ces compositions permet d'obtenir une action immédiate (telle que définie précédemment) et complémentaire dans le temps au niveau des mélanocytes tout en évitant également toute éventuelle interaction entre ces deux composés au sein d'une composition unique.

Encore plus avantageusement selon l'invention, la composition comprenant l'agoniste des récepteurs des prostaglandines est administrée en premier, puis la composition comprenant l'agoniste du récepteur MC1R est administrée en second.

De plus, la prise de ces compositions de façon décalée dans le temps peut permettre d'adapter la posologie de traitement en fonction de l'affection à traiter. Par exemple, la prise dans un premier temps de la composition comprenant l'agoniste des récepteurs des prostaglandines, puis la prise dans un second temps de la composition comprenant l'agoniste du récepteur MC1R de façon décalée offre un effet additionnel et synergique des deux composés au niveau de la zone à traiter et de la zone avoisinante, et plus particulièrement au niveau des mélanocytes initialement présents ou générés par le traitement, caractérisé par une repigmentation plus efficace de cette zone.

Selon un exemple de réalisation de l'invention, lorsque les composés sont présents au sein d'une même composition en vue d'une administration simultanée, ou lorsque les composés sont présents dans des compositions distinctes en vue d'une administration séparée, en même temps, la ou les compositions sont appliquées tous les jours.

Selon le mode de réalisation ou les composés sont présents au sein d'une même composition, ladite composition est une combinaison fixe et comprend dans un même véhicule physiologiquement acceptable (i) au moins un agoniste des récepteurs des prostaglandines et (ii) au moins un composé choisi parmi l'agoniste du récepteur MC1R et ses sels ou bases pharmaceutiquement acceptables. De préférence la composition est destinée à une seule application topique par jour.

Par véhicule physiologiquement acceptable on entend un support de formulation compatible avec la peau et les phanères.

Par combinaison fixe, il faut entendre une combinaison dont les principes actifs sont associés à des doses fixes au sein d'un seul et même véhicule (formule unique) les délivrant ensemble au point d'application.

Selon un autre exemple de réalisation de l'invention, lorsque les composés sont administrés dans des compositions distinctes en vue d'une administration étalée dans le temps, le régime d'administration de la composition comprenant l'agoniste des récepteurs des prostaglandines et de la composition comprenant l'agoniste du récepteur MC1R peut être variable. De manière préférentielle, la composition comprenant l'agoniste des récepteurs aux prostaglandines est administrée avant le traitement avec la composition comprenant l'agoniste du récepteur MC1R comme illustré dans l'exemple. Selon une manière préférée, elle est administrée sur les zones lésées pendant plusieurs jours consécutifs voire plusieurs semaines, puis la composition comprenant l'agoniste au récepteur MC1R est à son tour appliquée sur les zones lésées pendant plusieurs jours voire plusieurs semaines.

Le praticien veillera à adapter la durée du traitement en fonction de l'affection dermatologique liée à une hypopigmentation à traiter et du résultat recherché, le traitement pouvant s'étaler sur plusieurs mois.

De manière avantageuse, le produit selon l'invention comprend l'agoniste des récepteurs des prostaglandines à une concentration comprise entre environ 0,001 et 1% en poids, et, en particulier 0,004%, 0,005%, 0,01%, 0,03%, 0,04%, 0,05%, 0,1%, 0,2%, 0,5%, de préférence entre 0,005% et 0,1%, de manière particulièrement préférée 0,01% en poids, par rapport au poids total de la composition le comprenant.

L'agoniste du récepteur MC1R à une concentration comprise entre environ 0,001% et 15%, de manière préférée entre 0,001% et 10% en poids, de préférence entre 0,005% et 5%, de manière particulièrement préférée 5% en poids, mais aussi préférentiellement entre 0,01% et 9%, plus préférentiellement entre 0,1% et 8%, et en particulier à environ 0,005%, 0,01%, 0,05%, 0,1%, 0,5%, 1%, 5%,6%, 7%, 8%, 9% et 10%, en poids par rapport au poids total de la composition le comprenant.

Selon un aspect particulier de l'invention, la concentration de l'agoniste des récepteurs des prostaglandines à une concentration comprise entre environ 0.004% et 0.04% et la concentration de l'agoniste du récepteur MC1R à une concentration comprise entre environ 5% et 10% en poids par rapport au poids total de la composition le comprenant. Plus préférentiellement, la concentration de l'agoniste des récepteurs des prostaglandines à une concentration comprise entre environ 0.01% et 0.04% et la concentration de l'agoniste du récepteur MC1R à une concentration comprise entre environ 5% et 8% en poids par rapport au poids total de la composition.

Ainsi selon cet aspect particulier, l'invention se rapporte à un produit de combinaison comprenant au moins un agoniste des récepteurs des prostaglandines à une concentration comprise entre environ 0.004% et 0.04% et au moins un agoniste du récepteur MC1R à une concentration comprise entre environ 5% et 10% en poids par rapport au poids total de la composition le comprenant, comme médicament pour une utilisation simultanée, séparée ou étalée dans le temps pour le traitement et/ou la prévention des affections dermatologiques liées à une hypopigmentation.

La présente invention se rapporte donc à un produit de combinaison comme médicament destiné à être appliqué de manière topique ou par voie systémique de préférence par voie orale, la ou les compositions étant administrées par voie topique ou par voie orale, préférentiellement par voie topique.

Par voie orale, la ou les compositions peuvent se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie topique, la ou les compositions selon l'invention se présentent de préférence sous la forme d'onguents, crèmes, laits, pommades, poudres, tampons imbibés, syndets, solutions, gels, sprays, mousses, suspensions, sticks, shampoings, bases lavantes ou encore de suspensions de microsphères, nanosphères, vésicules lipidiques ou polymériques, patchs polymériques ou gélifiés permettant une libération contrôlée.

Les compositions de l'invention comprennent en outre un véhicule pharmaceutiquement acceptable, c'est-à-dire un véhicule adapté pour une utilisation en contact avec des cellules d'humains, sans toxicité, irritation, réponse allergique indue et similaires, et proportionné à un rapport avantage/risque raisonnable.

Il va maintenant être donné, à titre d'illustration et sans aucun caractère limitatif, plusieurs exemples de l'invention.

### Exemple :

### Mesure de l'activité propigmentante de la combinaison d'un agoniste des prostaglandines et en particulier PGF2α et de 2 agonistes du récepteur MC1R :

Chez les souris, les mélanocytes ne sont pas présents dans l'épiderme des zones couvertes de poils et sont principalement présents dans les follicules pileux. Les souris SKH2 ont la peau de la queue pigmentée, ce qui représente un intérêt particulier pour évaluer l'effet d'agents topiques sur la pigmentation de la peau. Les modifications de la pigmentation induites par ces composés topiques peuvent être quantifiées par différents moyens comme le score clinique de la pigmentation et la mesure de la couleur de la queue à l'aide d'un colorimètre.

L'évaluation de l'activité propigmentante du travoprost 0.01% seul ou en association avec des agonistes du récepteur MC1R: composé A à 7% ou composé B à 8%, est réalisée par application topique sur la queue de souris SKH2 pendant 57 jours. Le traitement est réalisé 5 jours par semaine pendant 57 jours. 20µl du produit à tester dilué dans le véhicule 145 sont appliqués sur la queue. Dans le cas des associations, 20µl de la solution de l'agoniste MC1R est appliqué après le traitement avec 20µl de la solution de travoprost 0.01%.

Le score clinique des animaux est réalisé 1 fois par semaine avant irradiation, la pigmentation est évaluée grâce à un score sur une échelle de 0 à 4. La répartition des scores est la suivante:
- 0 :: pigmentation naturelle
- 1 :: pigmentation légère
- 2 :: pigmentation modérée
- 3 :: pigmentation marquée
- 4 :: pigmentation intense

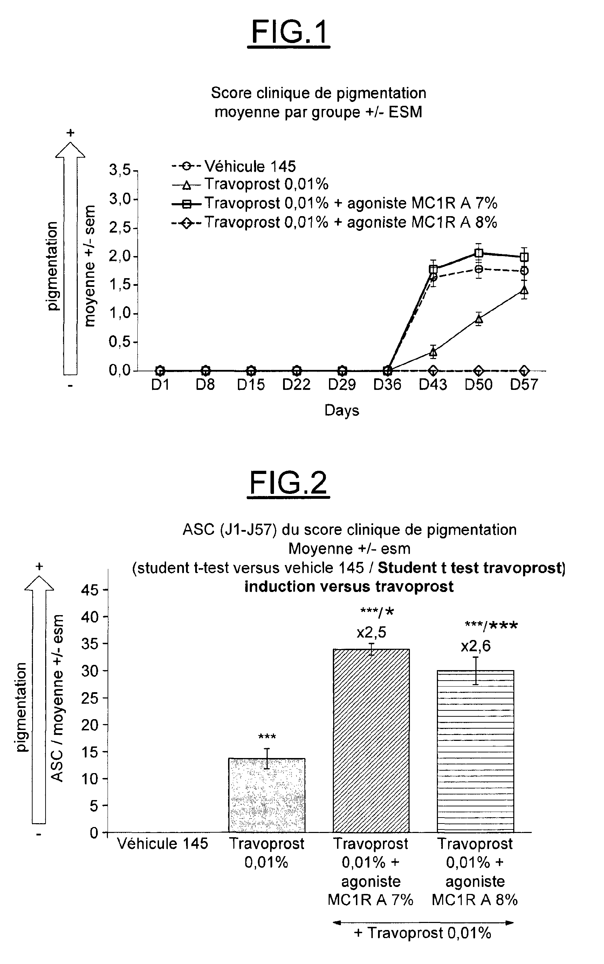

Les résultats de l'évaluation de la pigmentation et de l'analyse statistique sont représentés dans les figures 1 et 2:

### Score clinique de pigmentation

Les calculs suivants sont effectués :
- Moyenne ± esm des scores clinique par semaine par groupe de traitement (voir figure 1)
- Aires sous la courbe (AUC) des scores clinique par animal de J1 à J57 (voir figure 2).
- Index d'augmentation des AUC pour chaque association versus le groupe travoprost (voir figure 2).

L'AUC est obtenue en effectuant la somme des aires des rectangles entre le premier et le dernier jour de l'étude. Les différents groupes de traitements sont comparés au groupe véhicule et au groupe travoprost 0.01% sur ce paramètre au moyen d'un test t de Student.

Les résultats présentés dans les figures 1 et 2 montrent que le Travoprost seul à 0.01% augmente modérément la pigmentation naturelle de la peau de queue dès le jour 36 de l'étude par rapport au groupe traité par le véhicule. L'association de Travoprost 0.01% avec l'agoniste MC1R A à 7% ou l'agoniste MC1R B à 8%, augmente significativement la pigmentation naturelle de la peau par rapport au groupe traité par le véhicule et au groupe traité par Travoprost à 0.01% seul. Cet effet synergique est marqué avec les deux agonistes MC1R.

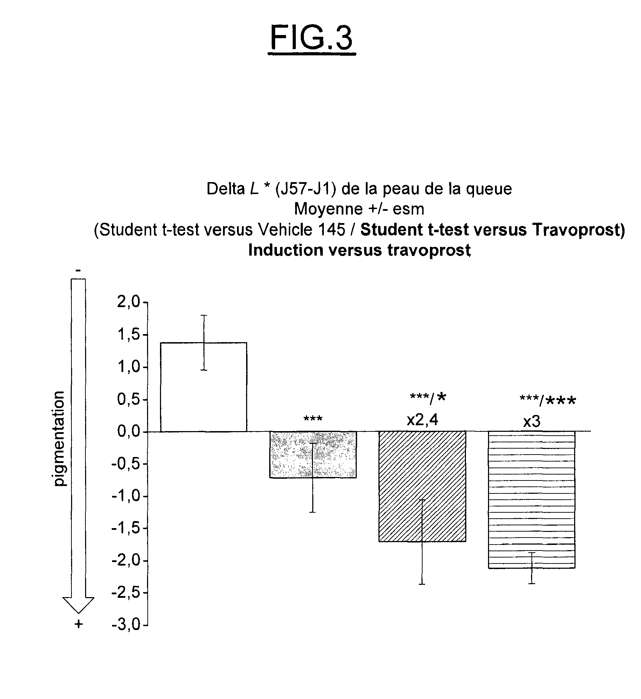

### Les résultats de mesure de la luminance sont présentés figure 3 :

Les calculs suivants sont effectués :
- Différence Moyenne ± esm de luminance (delta *L**) entre le début et la fin d'étude.
- Index d'augmentation du delta *L* pour chaque association versus le groupe Travoprost.

Les résultats présentés dans la figure 3 sur la différence de luminance de la peau entre le début et la fin d'étude confirment les résultats des scores cliniques de pigmentation et montrent un effet pro-pigmentant synergique de l'association de Travoprost 0.01% avec les agonistes MC1R A et B.

## Revendications

1. Produit de combinaison comprenant au moins un agoniste des récepteurs des prostaglandines PGF2α et au moins un agoniste du récepteur MC1R, comme médicament pour une utilisation simultanée, séparée ou étalée dans le temps pour le traitement et/ou la prévention des affections dermatologiques liées à une hypopigmentation.

2. Produit pour utilisation selon la revendication 1, pour le traitement et/ou la prévention des affections dermatologiques liées à une hypopigmentation choisies parmi le vitiligo, l'albinisme, les hypomélanoses, les dépigmentations par des agents physiques ou chimiques, les hypopigmentations postinflammatoires, le phénomène de Sutton ou toutes autres lésions hypopigmentaires.

3. Produit pour utilisation selon la revendication 2, pour le traitement et/ou la prévention du vitiligo.

4. Produit pour utilisation selon l'une des revendications 1 à 3, **caractérisé en ce que** l'agoniste des récepteurs des prostaglandines et l'agoniste du récepteur MC1R sont présents au sein d'une même composition.

5. Produit pour utilisation selon l'une des revendications 1 à 3, **caractérisé en ce que** l'agoniste des récepteurs des prostaglandines et l'agoniste du récepteur MC1R sont présents séparément l'un de l'autre au sein de compositions distinctes.

6. Produit pour utilisation selon la revendication 5, **caractérisé en ce que** la composition comprenant l'agoniste des récepteurs des prostaglandines est administrée en premier, puis la composition comprenant l'agoniste du récepteur MC1R est administrée en second.

7. Produit pour utilisation selon l'une des revendications 4 à 6, **caractérisé en ce que** l'agoniste des récepteurs des prostaglandines est présent à une concentration comprise entre environ 0,001 et 1% en poids, par rapport au poids total de la composition le comprenant.

8. Produit pour utilisation selon l'une des revendications 4 à 6, **caractérisé en ce que** l'agoniste du récepteur MC1R est présent à une concentration comprise entre environ 0,001% et 10% en poids par rapport au poids total de la composition le comprenant.

9. Produit pour utilisation selon les revendication 7 à 8 comprenant au moins un agoniste des récepteurs des prostaglandines à une concentration comprise entre environ 0.004% et 0.04% et au moins un agoniste du récepteur MC1R à une concentration comprise entre environ 5% et 10% en poids par rapport au poids total de la composition le comprenant, comme médicament pour une utilisation simultanée, séparée ou étalée dans le temps pour le traitement et/ou la prévention des affections dermatologiques liées à une hypopigmentation.

10. Produit pour utilisation selon l'une des revendications 4 à 9, **caractérisé en ce que** la ou les compositions sont administrées par voie topique.

11. Produit pour utilisation selon la revendication 10, **caractérisé en ce que** la ou les compositions se présentent sous la forme d'onguents, crèmes, laits, pommades, poudres, tampons imbibés, syndets, solutions, gels, sprays, mousses, suspensions, sticks, shampoings, bases lavantes ou encore de suspensions de microsphères, nanosphères, vésicules lipidiques ou polymériques, patchs polymériques ou gélifiés permettant une libération contrôlée.

12. Produit pour utilisation selon la revendication 1, **caractérisé en ce que** l'agoniste des récepteurs des prostaglandines est choisi parmi le latanoprost, le bimatoprost, le travoprost, le fluprostenol et l'unoprostone ; préférentiellement travoprost.

13. Produit pour utilisation selon l'une des revendications précédentes, **caractérisé en ce que** l'agoniste du récepteur MC1R est choisi parmi les hormones stimulantes du mélanocytes (MSH) α, β ou γ, l'hormone adrénocorticotropique (ACTH) 1-39, la β-lipotropine, la β-endorphine et des composés de formule générale (I)ou (II) suivante : 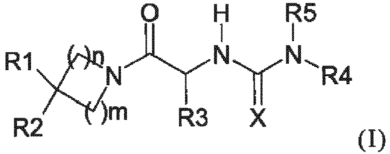 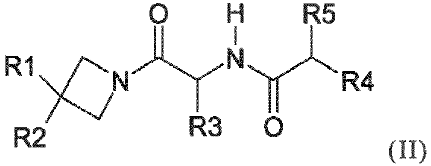 dans laquelle pour la formule générale (I):
R1 représente un atome d'hydrogène, un aryle, un aryle substitué, un alkyle, un cycloalkyle, un cycloalkylalkyle, ou un cycloalkylalkylalkyle ;
R2 représente un atome d'hydrogène, un hydroxy, un alkyle inférieur, un alkyle inférieur substitué, un alkyle supérieur, un alkyle supérieur substitué, un cycloalkyle, un cycloalkylalkyle, un alkoxy inférieur, un alkoxy inférieur substitué, un alkoxy supérieur, un alkoxy supérieur substitué, un cycloalkylalkoxy, un acyloxy, un acyle, un alkoxycarbonyl, un carboxamide, un acide carboxylique, un cyano, ou un amino disubstitué par un acyle et un aryle ou alkyle ;
R3 représente un aralkyle ou un aralkyle substitué ;
R4 représente un hétéroaralkyle ou un hétéroaralkyle substitué ;
R5 représente un atome d'hydrogène ou un alkyle ;
X représente un atome d'oxygène ou un atome de soufre ;
n, m peuvent être égaux à 1 ou 2 ;
ainsi que les sels et énantiomères correspondants.
Ou dans laquelle pour la formule (II) :
R1 représente un aryle, un aryle substitué ou un cycloalkyle ;
R2 représente un atome d'hydrogène, un hydroxy, un alkyle inférieur, un alkyle inférieur substitué, un alkyle supérieur, un alkyle supérieur substitué, un cycloalkyle, un cycloalkylalkyle pouvant être subsitué, un alkoxy inférieur, un alkoxy inférieur subsitué, un alkoxy supérieur, un alkoxy supérieur substitué, un cycloalkylalkoxy, ou un acyloxy ;
R3 représente un aralkyle ou un aralkyle substitué;
R4 représente un hétéroaralkyle, un hétéroaralkyle substitué, un hétéroalkyle ou un hétéroalkyle substitué;
R5 représente un atome d'hydrogène, un hydroxy, un amino, un acylamino ou un sulfonamide ;
ainsi que les sels et énantiomères correspondants.

14. Produit pour utilisation selon la revendication 13, **caractérisé en ce que** l'agoniste du récepteur MC1R est choisi parmi les composés de formule générale (II) dans laquelle:
R1 représente un cyclopropylmethyl ou un groupement 4-hydroxybutyl ;
R2 représente un atome d'hydrogène, un groupement méthyl.

15. Produit pour utilisation selon la revendication 13, **caractérisé en ce que** l'agoniste du récepteur MC1R est choisi parmi :
1-[(S)-2-(4-Butyryl-4-phényl-pipéridin-1-yl)-1-(4-méthoxy-benzyl)-2-oxo-éthyl]-3-[2-(1H-imidazol-4-yl)-éthyl]-urée ;
1-[2-(1H-Imidazol-4-yl}-éthyl]-3-[1-(4-méthoxy-benzyl)-2-oxo-2-(4-oxo-1-phényl-1,3,8-triaza-spiro[4.5]déc-8-yl)-éthyl]-urée ;
1-[2-(4-Cyano-4-phényl-pipéridin-1-yl)-1-(4-méthoxy-benzyl)-2-oxo-éthyl]-3-[2-(1H-imidazol-4-yl)-éthyl]-urée ;
1-[2-(1H-Imidazol-4-yl)-éthyl]-3-[1-(4-méthoxy-benzyl)-2-oxo-2-(4-phényl-pipéridin-1-yl)-éthyl]-urée ;
1-[2-(1H-Imidazol-4-yl)-éthyl]-3-[1-(4-méthoxy-benzyl)-2-oxo-2-pipéridin-1-yl-éthyl]-urée ;
4-Cyclohexyl-1-[2-{3-[2-(1H-imidazol-4-yl)-éthyl]-uréido}-3-(4-méthoxy-phényl)-propionyl]-pipéridine-4-carboxylate d'éthyle ;
N-{1-[2-{3-[2-(1H-Imidazol-4-yl)-éthyl]-uréido}-3-(4-méthoxy-phényl)-propionyl]-pipéridin-4-yl}-N-phényl-propionamide ;
Butyrate de 1-[2-{3-[2-(1H-imidazol-4-yl)-éthyl]-uréido}-3-(4-méthoxy-phényl)-propionyl]-3-phényl-azétidin-3-yle ;
1-[2-{3-[2-(1H-Imidazol-4-yl)-éthyl]-uréido}-3-(4-méthoxy-phényl)-propionyl]-pipéridine-4-carboxylate d'éthyle ;
1-[2-(1H-Imidazol-4-yl)-éthyl]-3-{1-(4-méthoxy-benzyl)-2-[4-(2-méthoxy-phényl)-pipéridin-1-yl]-2-oxo-éthyl}-urée ;
1-[2-{3-Butoxy-3-phényl-azétidin-1-yl)-1-(4-méthoxy-benzyl)-2-oxo-éthyl]-3-[2-(1H-imidazol-4-yl)-éthyl]-urée ;
4-Cyclohexyl-1-[2-{3-[2-(1H-imidazol-4-yl)-éthyl]-uréido}-3-(4-méthoxy-phényl)-propionyl]-pipéridine-4-carboxylamide de méthyle ;
1-[2-(3-Cyclohexanecarbonyl-azétidin-1-yl)-1-(4-méthoxy-benzyl)-2-oxo-éthyl]-3-[2-(1H-imidazol-4-yl)-éthyl]-urée ;
4-Cyclohexyl-1-[2-{3-éthyl-3-[2-(1H-imidazol-4-yl)-éthyl]-uréido}-3-(4-méthoxy-phényl)-propionyl]-pipéridine-4-carboxylate d'éthyle ;
N-Cyclopropyl-N-{1-[2-{3-[2-(1H-imidazol-4-yl)-éthyl]-uréido}-3-(4-méthoxy-phényl)-propionyl]-pipéridin-4-yl}-propionamide ;
4-Cyclohexyl-1-(2-{3-[2-(1H-imidazol-4-yl)-éthyl]-uréido}-3-phényl-propionyl)-pipéridine-4-carboxylate d'éthyle ;
1-[2-(4-Butyryl-4-cyclohexyl-pipéridin-1-yl)-1-(4-méthoxy-benzyl)-2-oxo-éthyl]-3-[2-(3H-imidazol-4-yl)-éthyl]-urée ;
1-[2-(4-Butoxy-4-cyclohexyl-pipéridin-1-yl)-1-(4-méthoxy-benzyl)-2-oxo-éthyl]-3-[2-(1H-imidazol-4-yl)-éthyl]-urée ;
4-Cyclohexyl-1-(2-{3-[2-(1H-imidazol-4-yl)-éthyl]-uréido}-2-phényl-acétyl)-pipéridine-4-carboxylate d'éthyle ;
4-Cyclohexyl-1-[2-{3-[2-(1H-imidazol-4-yl)-éthyl]-uréido}-3-(4-méthoxy-phényl)-propionyl]-pipéridine-4-carboxylate de méthyle ;
1-[2-(4-Cyclohexyl-4-éthoxy-pipéridin-1-yl)-1-(4-méthoxy-benzyl)-2-oxo-éthyl]-3-[2-(1H-imidazol-4-yl)-éthyl]-urée ;
1-[2-(4-Acétyl-4-cyclohexyl-pipéridin-1-yl)-1-(4-méthoxy-benzyl)-2-oxo-éthyl]-3-[2-(1H-imidazol-4-yl)-éthyl]-urée
4-Cyclohexyl-1-(2-{3-[2-(1H-imidazol-4-yl)-éthyl]-uréido}-2-phényl-acétyl)-pipéridine-4-carboxylate de méthyle ;
4-Ethyl-1-[2-{3-[2-(1H-imidazol-4-yl)-éthyl]-uréido}-3-(4-méthoxy-phényl)-propionyl]-pipéridine-4-carboxylate d'éthyle ;
1-[2-(4-Cyclohexyl-4-propoxy-pipéridin-1-yl)-1-(4-méthoxy-benzyl)-2-oxo-éthyl]-3-[2-(1H-imidazol-4-yl)-éthyl]-urée ;
Acide 4-cyclohexyl-1-[2-{3-[2-(1H-imidazol-4-yl)-éthyl]-uréido}-3-(4-méthoxy-phényl)-propionyl]-pipéridine-4-carboxylique ;
1-[2-(1H-Imidazol-4-yl)-éthyl]-3-{1-(4-méthoxy-benzyl)-2-[3-(2-méthyl-cyclohexyl)-3-propoxy-azétidin-1-yl]-2-oxo-éthyl}-urée ;
4-Cyclohexyl-1-[2-{3-[2-(1H-imidazol-4-yl)-éthyl]-uréido}-3-(4-méthoxy-phényl)-propionyl]-pipéridine-4-carboxylate de propyle ;
1-[2-(1H-Imidazol-4-yl)-éthyl]-3-[1-(4-méthoxy-benzyl)-2-oxo-2-(3-pentyl-3-phényl-azétidin-1-yl)-éthyl]-urée ;
1-((R)-3-(4-Chloro-phényl)-2-{3-[2-(1H-imidazol-4-yl)-éthyl]-uréido}-propionyl)-4-cyclohexyl-pipéridine-4-carboxylate d'éthyle ;
1-((S)-3-(4-Chloro-phényl)-2-{3-[2-(1H-imidazol-4-yl)-éthyl]-uréido}-propionyl)-4-cyclohexyl-pipéridine-4-carboxylate d'éthyle ;
1-[2-(4-Cyclohexyl-4-propionyl-pipéridin-1-yl)-1-(4-méthoxy-benzyl)-2-oxo-éthyl]-3-[2-(1H-imidazol-4-yl)-éthyl]-urée ;
1-[2-(4-Cyclohexyl-4-propionyl-pipéridin-1-yl)-1-(4-méthoxy-benzyl)-2-oxo-éthyl]-3-(1H-imidazol-4-ylméthyl)-urée ;
4-Cyclohexyl-1-[(R)-2-{3-[2-(1H-imidazol-4-yl)-éthyl]-uréido}-3-(4-méthoxy-phényl)-propionyl]-pipéridine-4-carboxylate d'éthyle ;
4-Cyclopropylméthyl-1-[2-{3-[2-(1H-imidazol-4-yl)-éthyl]-uréido}-3-(4-méthoxy-phényl}-propionyl]-pipéridine-4-carboxylate d'éthyle ;
4-Cyclohexyl-1-(2-{3-[2-(1H-imidazol-4-yl)-éthyl]-uréido}-3-phényl-propionyl)-pipéridine-4-carboxylate de propyle ;
4-Cyclopentyl-1-(2-{3-[2-(1H-imidazol-4-yl)-éthyl]-uréido}-3-phényl-propionyl)-pipéridine-4-carboxylate d'éthyle ;
4-Cyclopentyl-1-[2-{3-[2-(1H-imidazol-4-yl)-éthyl]-uréido}-3-(4-méthoxy-phényl)-propionyl]-pipéridine-4-carboxylate d'éthyle ;
4-Cyclohexyl-1-[(S)-2-{3-[2-(1H-imidazol-4-yl)-éthyl]-uréido}-3-(4-méthoxy-phényl)-propionyl]-pipéridine-4-carboxylate d'éthyle ;
1-[(R)-2-(4-Butyryl-4-cyclohexyl-pipéridin-1-yl)-1-(4-méthoxy-benzyl)-2-oxo-éthyl]-3-[2-(1H-imidazol-4-yl)-éthyl]-urée ;
1-[(R)-2-(4-Butyryl-4-cyclohexyl-pipéridin-1-yl)-1-(4-fluoro-benzyl)-2-oxo-éthyl]-3-[2-(1H-imidazol-4-yl)-éthyl]-urée ;
1-[(R)-1-Benzyl-2-(4-butyryl-4-cyclohexyl-pipéridin-1-yl)-2-oxo-éthyl]-3-[2-(1H-imidazol-4-yl)-éthyl]-urée ;
1-[(R)-2-(4-Butyryl-4-cyclohexyl-pipéridin-1-yl)-1-(4-methoxy-benzyl)-2-oxo-éthyl]-3-[2-(3-méthyl-3H-imidazol-4-yl)-éthyl]-urée;
1-[(R)-2-(4-Butyryl-4-cyclohexyl-pipéridin-1-yl)-1-(4-chloro-benzyl)-2-oxo-éthyl]-3-[2-(1H-imidazol-4-yl)-éthyl]-urée ;
4-Cyclohexyl-1-((R)-3-(3,4-dichloro-phényl)-2-{3-[3-(1H-imidazol-4-yl)-propyl]-uréido}-propionyl)-pipéridine-4-carboxylate d'éthyle ;
4-Cyclohexyl-1-((R)-3-(4-méthoxy-phényl)-2-{3-[2-(3-méthyl-3H-imidazol-4-yl)-éthyl]-uréido}-propionyl)-pipéridine-4-carboxylate d'éthyle ;
4-Cyclohexyl-1-[(R)-2-{3-[2-(1H-imidazol-4-yl)-éthyl]-thiouréido}-3-(4-méthoxy-phényl)-propionyl]-pipéridine-4-carboxylate d'éthyle ;
1-[(R)-2-(4-Butyryl-4-cyclohexyl-pipéridin-1-yl)-1-(4-méthoxy-benzyl)-2-oxo-éthyl]-3-[2-(1H-imidazol-4-yl)-éthyl]-thiourée ;
1-[(R)-2-(4-Cyclohexyl-4-propoxy-pipéridin-1-yl)-1-(4-méthoxy-benzyl)-2-oxo-éthyl]-3-[2-(1H-imidazol-4-yl)-éthyl]-thiourée ;
1-[(R)-1-Benzyl-2-(4-cyclohexyl-4-propoxy-pipéridin-1-yl)-2-oxo-éthyl]-3-[2-(1H-imidazol-4-yl)-éthyl]-thiourée ;
1-[(R)-1-Benzyl-2-(4-cyclohexyl-4-propoxy-pipéridin-1-yl)-2-oxo-éthyl]-3-[2-(1H-imidazol-4-yl)-éthyl]-urée ;
4-Cyclohexyl-1-((R)-3-(4-méthoxy-phényl)-2-{3-[2-(3-méthyl-3H-imidazol-4-yl)-éthyl]-thiouréido}-propionyl)-pipéridine-4-carboxylate d'éthyle ;
4-Cyclohexyl-1-((R)-2-{3-[2-(3-méthyl-3H-imidazol-4-yl)-éthyl]-uréido}-3-phényl-propionyl)-pipéridine-4-carboxylate d'éthyle ;
1-[(R)-2-(4-Cyclohexyl-4-propoxy-pipéridin-1-yl)-1-(4-méthoxy-benzyl)-2-oxo-éthyl]-3-[2-(3-méthyl-3H-imidazol-4-yl)-éthyl]-urée ;
1-((R)-3-(4-Chloro-phényl)-2-{3-[2-(3-méthyl-3H-imidazol-4-yl)-éthyl]-uréido}-propionyl)-4-cyclohexyl-pipéridine-4-carboxylate d'éthyle ;
4-Cyclohexyl-1-((R)-3-(4-fluoro-phényl)-2-{3-[2-(3-méthyl-3H-imidazol-4-yl)-éthyl]-uréido}-propionyl)-pipéridine-4-carboxylate d'éthyle ;
4-Cyclohexyl-1-((R)-3-(4-fluoro-phényl)-2-{3-[2-(1H-imidazol-4-yl)-éthyl]-uréido}-propionyl)-pipéridine-4-carboxylate d'éthyle ;
4-Cyclohexyl-1-((R)-3-(4-fluoro-phényl)-2-{3-[2-(1H-imidazol-4-yl)-éthyl]-thiouréido}-propionyl)-pipéridine-4-carboxylate d'éthyle ;
1-((R)-3-(4-Chloro-phényl)-2-{3-[2-(1H-imidazol-4-yl)-éthyl]-thiouréido}-propionyl)-4-cyclohexyl-pipéridine-4-carboxylate d'éthyle ;
1-((R)-3-(4-Chloro-phényl)-2-{3-[2-(3-méthyl-3H-imidazol-4-yl)-éthyl]-thiouréido}-propionyl)-4-cyclohexyl-pipéridine-4-carboxylate d'éthyle ;
1-[(R)-2-(4-Cyclohexyl-4-propoxy-pipéridin-1-yl)-1-(4-fluoro-benzyl)-2-oxo-éthyl]-3-[2-(1H-imidazol-4-yl)-éthyl]-urée ;
1-[(R)-1-(4-Chloro-benzyl)-2-(4-cyclohexyl-4-propoxy-pipéridin-1-yl)-2-oxo-éthyl]-3-[2-(1H-imidazol-4-yl)-éthyl]-urée ;
N-[(R)-2-[3-(4-Hydroxy-butoxy)-3-o-tolyl-azetidin-1-yl]-1-(4-methoxy-benzyl)-2-oxo-ethyl]-3-(-1H-imidazol-4-yl)-propionamide.
N-[(R)-2-(3-Cyclopropylmethoxy-3-o-tolyl-azetidin-1-yl)-1-(4-methoxy-benzyl)-2-oxo-ethyl]-3-(5-methyl-1H-imidazol-4-yl)-propionamide
ainsi que leurs sels et énantiomères respectifs.

16. Produit pour utilisation selon la revendication 14, **caractérisé en ce que** les agonistes du récepteur MC1R sont choisi parmi **:**
4-Cyclohexyl-1-[(R)-2-{3-[2-(1H-imidazol-4-yl)-éthyl]-uréido}-3-(4-méthoxy-phényl)-propionyl]-pipéridine-4-carboxylate d'éthyle.
N-[(R)-2-[3-(4-Hydroxy-butoxy)-3-o-tolyl-azetidin-1-yl]-1-(4-methoxy-benzyl)-2-oxo-ethyl]-3-(-1H-imidazol-4-yl)-propionamide.
N-[(R)-2-(3-Cyclopropylmethoxy-3-o-tolyl-azetidin-1-yl)-1-(4-methoxy-benzyl)-2-oxo-ethyl]-3-(5-methyl-lH-imidazol-4-yl)-propionamide
ainsi que les sels et énantiomères correspondants.

17. Un agoniste du récepteur MC1R pour une utilisation dans le traitement et/ou la prévention des affections dermatologiques liées à une hypopigmentation, en association ou en combinaison avec au moins un agoniste des récepteurs des prostaglandines PGF2α, comme médicament pour une utilisation simultanée, séparée ou étalée dans le temps.

## Patentansprüche

1. Kombinationsprodukt, umfassend mindestens einen Agonisten von PGF2α-Prostaglandinrezeptoren und mindestens einen Agonisten des Rezeptors MC1R als Arzneimittel für eine gleichzeitige, getrennte oder zeitlich verteilte Verwendung zur Behandlung und/oder Vorbeugung dermatologischer Krankheiten, die mit einer Hypopigmentierung einhergehen.

2. Produkt für die Verwendung nach Anspruch 1 zur Behandlung und/oder Vorbeugung dermatologischer Krankheiten, die mit einer Hypopigmentierung einhergehen, ausgewählt aus Vitiligo, Albinismus, Hypomelanosen, Depigmentierungen durch physikalische oder chemische Mittel, postinflammatorischen Hypopigmentierungen, Sutton-Phänomen oder allen anderen Hypopigmentierungsläsionen.

3. Produkt für die Verwendung nach Anspruch 2 zur Behandlung und/oder Vorbeugung von Vitiligo.

4. Produkt für die Verwendung nach einem der Ansprüche 1 bis 3, **dadurch gekennzeichnet, dass** der Agonist von Prostaglandinrezeptoren und der Agonist des Rezeptors MC1R in derselben Zusammensetzung vorhanden sind.

5. Produkt für die Verwendung nach einem der Ansprüche 1 bis 3, **dadurch gekennzeichnet, dass** der Agonist von Prostaglandinrezeptoren und der Agonist des Rezeptors MC1R getrennt voneinander in separaten Zusammensetzungen vorhanden sind.

6. Produkt für die Verwendung nach Anspruch 5, **dadurch gekennzeichnet, dass** die Zusammensetzung, die den Agonisten von Prostaglandinrezeptoren umfasst, als erste verabreicht wird, dann die Zusammensetzung, die den Agonisten des Rezeptors MC1R umfasst, als zweite verabreicht wird.

7. Produkt für die Verwendung nach einem der Ansprüche 4 bis 6, **dadurch gekennzeichnet, dass** der Agonist von Prostaglandinrezeptoren in einer Konzentration zwischen etwa 0,001 und 1 Gew.-%, bezogen auf das Gesamtgewicht der Zusammensetzung, die ihn umfasst, vorhanden ist.

8. Produkt für die Verwendung nach einem der Ansprüche 4 bis 6, **dadurch gekennzeichnet, dass** der Agonist des Rezeptors MC1R in einer Konzentration zwischen etwa 0,001 Gew.-% und 10 Gew.-%, bezogen auf das Gesamtgewicht der Zusammensetzung, die ihn umfasst, vorhanden ist.

9. Produkt für die Verwendung nach den Ansprüchen 7 bis 8, umfassend mindestens einen Agonisten von Prostaglandinrezeptoren in einer Konzentration zwischen etwa 0,004% und 0,04% und mindestens einen Agonisten des Rezeptors MC1R in einer Konzentration zwischen etwa 5 Gew.-% und 10 Gew.-%, bezogen auf das Gesamtgewicht der Zusammensetzung, die ihn umfasst, als Arzneimittel für eine gleichzeitige, getrennte oder über die Zeit verteilte Verwendung zur Behandlung und/oder Vorbeugung dermatologischer Krankheiten, die mit einer Hypopigmentierung einhergehen.

10. Produkt für die Verwendung nach einem der Ansprüche 4 bis 9, **dadurch gekennzeichnet, dass** die Zusammensetzung oder die Zusammensetzungen topisch verabreicht wird/werden.

11. Produkt für die Verwendung nach Anspruch 10, **dadurch gekennzeichnet, dass** die Zusammensetzung oder die Zusammensetzungen in Form von Salben, Cremes, Milch, Wundsalben, Pulvern, getränkten Bauschen, Syndets, Lösungen, Gelen, Sprays, Schäumen, Suspensionen, Stiften, Shampoos, Waschmittelbasen oder auch Suspensionen von Mikrosphären, Nanosphären, Lipid- oder Polymervesikeln, polymeren oder gelierten Pflastern, die eine kontrollierte Freisetzung gestatten, dargereicht wird/werden.

12. Produkt für die Verwendung nach Anspruch 1, **dadurch gekennzeichnet, dass** der Agonist von Prostaglandinrezeptoren aus Latanoprost, Bimatoprost, Travoprost, Fluprostenol und Unoproston, vorzugsweise Travoprost, ausgewählt ist.

13. Produkt für die Verwendung nach einem der vorhergehenden Ansprüche, **dadurch gekennzeichnet, dass** der Agonist des Rezeptors MC1R ausgewählt ist aus den Melanozyten-stimulierenden Hormonen (MSH) α, β oder γ, dem adrenocorticotropen Hormon (ACTH) 1-39, β-Lipotropin, β-Endorphin und Verbindungen der folgenden allgemeinen Formel (I) oder (II): 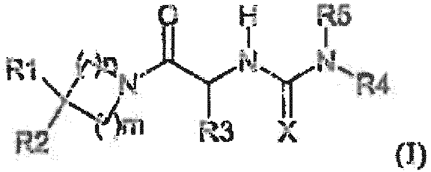 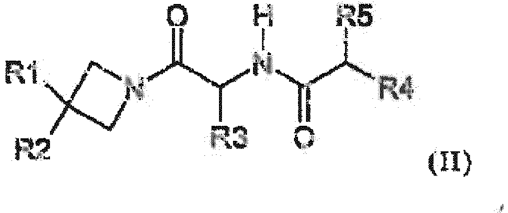 wobei für die allgemeine Formel (I):
R1 ein Wasserstoffatom, ein Aryl, ein substituiertes Aryl, ein Alkyl, ein Cycloalkyl, ein Cycloalkylalkyl oder ein Cycloalkylalkylalkyl darstellt;
R2 ein Wasserstoffatom, ein Hydroxy, ein Niederalkyl, ein substituiertes Niederalkyl, ein höheres Alkyl, ein substituiertes höheres Alkyl, ein Cycloalkyl, ein Cycloalkylalkyl, ein Niederalkoxy, ein substituiertes Niederalkoxy, ein höheres Alkoxy, ein substituiertes höheres Alkoxy, ein Cycloalkylalkoxy, ein Acyloxy, ein Acyl, ein Alkoxycarbonyl, ein Carboxamid, eine Carbonsäure, ein Cyano oder ein mit einem Acyl und einem Aryl oder Alkyl disubstituiertes Amino darstellt;
R3 ein Aralkyl oder ein substituiertes Aralkyl darstellt;
R4 ein Heteroaralkyl oder ein substituiertes Heteroaralkyl darstellt;
R5 ein Wasserstoffatom oder ein Alkyl darstellt;
X ein Sauerstoffatom oder ein Schwefelatom darstellt;
n, m gleich 1 oder 2 sein können;
sowie den entsprechenden Salzen und Enantiomeren.
Oder wobei in der allgemeinen Formel (II):
R1 ein Aryl, ein substituiertes Aryl oder ein Cycloalkyl darstellt;
R2 ein Wasserstoffatom, ein Hydroxy, ein Niederalkyl, ein substituiertes Niederalkyl, ein höheres Alkyl, ein substituiertes höheres Alkyl, ein Cycloalkyl, ein Cycloalkylalkyl, das substituiert sein kann, ein Niederalkoxy, ein substituiertes Niederalkoxy, ein höheres Alkoxy, ein substituiertes höheres Alkoxy, ein Cycloalkylalkoxy oder ein Acyloxy darstellt;
R3 ein Aralkyl oder ein substituiertes Aralkyl darstellt;
R4 ein Heteroaralkyl, ein substituiertes Heteroaralkyl, ein Heteroalkyl oder ein substituiertes Heteroalkyl darstellt;
R5 ein Wasserstoffatom, ein Hydroxy, ein Amino, ein Acylamino oder ein Sulfonamid darstellt;
sowie den entsprechenden Salzen und Enantiomeren.

14. Produkt für die Verwendung nach Anspruch 13, **dadurch gekennzeichnet, dass** der Agonist des Rezeptors MC1R aus Verbindungen der allgemeinen Formel (II) ausgewählt ist, wobei:
R1 ein Cyclopropylmethyl oder eine 4-Hydroxybutylgruppe darstellt;
R2 ein Wasserstoffatom, eine Methylgruppe darstellt.

15. Produkt für die Verwendung nach Anspruch 13, **dadurch gekennzeichnet, dass** der Agonist des Rezeptors MC1R aus Folgenden ausgewählt ist:
1-[(S)-2-(4-Butyryl-4-phenylpiperidin-1-yl)-1-(4-methoxybenzyl)-2-oxoethyl]-3-[2-(1H-imidazol-4-yl)-ethyl]-harnstoff;
1-[2-(1H-Imidazol-4-yl)-ethyl]-3-[1-(4-methoxybenzyl)-2-oxo-2-(4-oxo-1-phenyl-1,3,8-triazaspiro[4.5]dec-8-yl)-ethyl]-harnstoff;
1-[2-(4-Cyano-4-phenylpiperidin-1-yl)-1-(4-methoxybenzyl)-2-oxoethyl]-3-[2-(1H-imidazol-4-yl)-ethyl]-harnstoff;
1-[2-(1H-Imidazol-4-yl)-ethyl]-3-[1-(4-methoxybenzyl)-2-oxo-2-(4-phenylpiperidin-1-yl)-ethyl]-harnstoff;
1-[2-(1H-Imidazol-4-yl)-ethyl]-3-[1-(4-methoxybenzyl)-2-oxo-2-piperidin-1-yl-ethyl]-harnstoff;
Ethyl-4-cyclohexyl-1-[2-{3-[2-(1H-imidazol-4-yl)-ethyl]-ureido}-3-(4-methoxyphenyl)-propionyl]-piperidin-4-carboxylat;
N-{1-[2-{3-[2-(1H-Imidazol-4-yl)-ethyl]-ureido}-3-(4-methoxyphenyl)-propionyl]-piperidin-4-yl}-N-phenylpropionamid;
1-[2-{3-[2-(1H-Imidazol-4-yl)-ethyl]-ureido}-3-(4-methoxyphenyl)-propionyl]-3-phenylazetidin-3-ylbutyrat;
Ethyl-1-[2-{3-[2-(1H-imidazol-4-yl)-ethyl]-ureido}-3-(4-methoxyphenyl)propionyl]piperidin-4-carboxylat;
1-[2-(1H-Imidazol-4-yl)-ethyl]-3-{1-(4-methoxybenzyl)-2-[4-(2-methoxyphenyl)-piperidin-1-yl]-2-oxoethyl}-harnstoff;
1-[2-(3-Butoxy-3-phenylazetidin-1-yl)-1-(4-methoxybenzyl)-2-oxoethyl]-3-[2-(1H-imidazol-4-yl)-ethyl]-harnstoff;
Methyl-4-cyclohexyl-1-[2-{3-[2-(1H-imidazol-4-yl)-ethyl]-ureido}-3-(4-methoxyphenyl)-propionyl]-piperidin-4-carbonsäureamid;
1-[2-(3-Cyclohexancarbonylazetidin-1-yl)-1-(4-methoxybenzyl)-2-oxoethyl]-3-[2-(imidazol-4-yl)-ethyl]-harnstoff;
Ethyl-4-cyclohexyl-1-[2-{3-ethyl-3-[2-(1H-imidazol-4-yl)-ethyl]-ureido}-3-(4-methoxyphenyl)-propionyl]-piperidin-4-carboxylat;
N-Cyclopropyl-N-{1-[2-{3-[2-(1H-imidazol-4-yl)-ethyl]-ureido}-3-(4-methoxyphenyl)-propionyl]-piperidin-4-yl}-propionamid;
Ethyl-4-cyclohexyl-1-(2-{3-[2-(1H-imidazol-4-yl)-ethyl]-ureido}-3-phenylpropionyl)-piperidin-4-carboxylat;
1-[2-(4-Butyryl-4-cyclohexylpiperidin-1-yl)-1-(4-methoxybenzyl)-2-oxoethyl]-3-[2-(3H-imidazol-4-yl)-ethyl]-harnstoff;
1-[2-(4-Butoxy-4-cyclohexylpiperidin-1-yl)-1-(4-methoxybenzyl)-2-oxoethyl]-3-[2-(1H-imidazol-4-yl)-ethyl]-harnstoff;
Ethyl-4-cyclohexyl-1-(2-{3-[2-(1H-imidazol-4-yl)-ethyl]-ureido}-2-phenylacetyl)piperidin-4-carboxylat;
Methyl-4-cyclohexyl-1-[2-{3-[2-(1H-imidazol-4-yl)-ethyl]-ureido}-3-(4-methoxyphenyl)-propionyl]-piperidin-4-carboxylat;
1-[2-(4-Cyclohexyl-4-ethoxypiperidin-1-yl)-1-(4-methoxybenzyl)-2-oxoethyl]-3-[2-(1H-imidazol-4-yl)-ethyl]-harnstoff;
1-[2-(4-Acetyl-4-cyclohexylpiperidin-1-yl)-1-(4-methoxybenzyl)-2-oxoethyl]-3-[2-(1H-imidazol-4-yl)-ethyl]-harnstoff;
Methyl-4-cyclohexyl-1-(2-{3-[2-(1H-imidazol-4-yl)-ethyl]-ureido}-2-phenylacetyl)-piperidin-4-carboxylat;
Ethyl-4-ethyl-1-[2-{3-[2-(1H-imidazol-4-yl)-ethyl]-ureido}-3-(4-methoxyphenyl)-propionyl]-piperidin-4-carboxylat;
1-[2-(4-Cyclohexyl-4-propoxypiperidin-1-yl)-1-(4-methoxybenzyl)-2-oxoethyl]-3-[2-(1H-imidazol-4-yl)-ethyl]-harnstoff;
4-Cyclohexyl-1-[2-{3-[2-(1H-imidazol-4-yl)-ethyl]-ureido}-3-(4-methoxyphenyl)-propionyl]-piperidin-4-carbonsäure;
1-[2-(1H-Imidazol-4-yl)-ethyl]-3-{1-(4-methoxybenzyl)-2-[3-(2-methylcyclohexyl)-3-propoxyazetidin-1-yl]-2-oxoethyl}-harnstoff;
Propyl-4-cyclohexyl-1-[2-{3-[2-(1H-imidazol-4-yl)-ethyl]-ureido}-3-(4-methoxyphenyl)-propionyl]-piperidin-4-carboxylat;
1-[2-(1H-Imidazol-4-yl)-ethyl]-3-[1-(4-methoxybenzyl)-2-oxo-2-(3-pentyl-3-phenylazetidin-1-yl)-ethyl]-harnstoff;
Ethyl-1-((R)-3-(4-chlorphenyl)-2-{3-[2-(1H-imidazol-4-yl)-ethyl]-ureido}-propionyl)-4-cyclohexylpiperidin-4-carboxylat;
Ethyl-1-((S)-3-(4-chlorphenyl)-2-{3-[2-(1H-imidazol-4-yl)-ethyl]-ureido}-propionyl)-4-cyclohexylpiperidin-4-carboxylat;
1-[2-(4-Cyclohexyl-4-propionylpiperidin-1-yl)-1-(4-methoxybenzyl)-2-oxoethyl]-3-[2-(1H-imidazol-4-yl)-ethyl]-harnstoff;
1-[2-(4-Cyclohexyl-4-propionylpiperidin-1-yl)-1-(4-methoxybenzyl)-2-oxoethyl]-3-(1H-imidazol-4-ylmethyl)-harnstoff;
Ethyl-4-cyclohexyl-1-[(R)-2-{3-[2-(1H-imidazol-4-yl)-ethyl]-ureido}-3-(4-methoxyphenyl)-propionyl]-piperidin-4-carboxylat;
Ethyl-4-cyclopropylmethyl-1-[2-{3-[2-(1H-imidazol-4-yl)-ethyl]-ureido}-3-(4-methoxyphenyl)-propionyl]-piperidin-4-carboxylat;
Propyl-4-cyclohexyl-1-(2-{3-[2-(1H-imidazol-4-yl)-ethyl]-ureido}-3-phenylpropionyl)-piperidin-4-carboxylat;
Ethyl-4-cyclopentyl-1-(2-{3-[2-(1H-imidazol-4-yl)-ethyl]-ureido}-3-phenylpropionyl)-piperidin-4-carboxylat;
Ethyl-4-cyclopentyl-1-[2-{3-[2-(1H-imidazol-4-yl)-ethyl]-ureido}-3-(4-methoxyphenyl)-propionyl]-piperidin-4-carboxylat;
Ethyl-4-cyclohexyl-1-[(S)-2-{3-[2-(1H-imidazol-4-yl)-ethyl]-ureido}-3-(4-methoxyphenyl)-propionyl]-piperidin-4-carboxylat;
1-[(R)-2-(4-Butyryl-4-cyclohexylpiperidin-1-yl)-1-(4-methoxybenzyl)-2-oxoethyl]-3-[2-(1H-imidazol-4-yl)-ethyl]-harnstoff;
1-[(R)-2-(4-Butyryl-4-cyclohexylpiperidin-1-yl)-1-(4-fluorbenzyl)-2-oxoethyl]-3-[2-(1H-imidazol-4-yl)-ethyl]-harnstoff;
1-[(R)-1-Benzyl-2-(4-butyryl-4-cyclohexylpiperidin-1-yl)-2-oxoethyl]-3-[2-(1H-imidazol-4-yl)-ethyl]-harnstoff;
1-[(R)-2-(4-Butyryl-4-cyclohexylpiperidin-1-yl)-1-(4-methoxybenzyl)-2-oxoethyl]-3-[2-(3-methyl-3H-imidazol-4-yl)-ethyl]-harnstoff;
1-[(R)-2-(4-Butyryl-4-cyclohexylpiperidin-1-yl)-1-(4-chlorbenzyl)-2-oxoethyl]-3-[2-(1H-imidazol-4-yl)-ethyl]-harnstoff;
Ethyl-4-cyclohexyl-1-((R)-3-(3,4-dichlorphenyl)-2-{3-[3-(1H-imidazol-4-yl)-propyl]-ureido}-propionyl)-piperidin-4-carboxylat;
Ethyl-4-cyclohexyl-1-((R)-3-(4-methoxyphenyl)-2-{3-[2-(3-methyl-3H-imidazol-4-yl)-ethyl]-ureido}-propionyl)-piperidin-4-carboxylat;
Ethyl-4-cyclohexyl-1-[(R)-2-{3-[2-(1H-imidazol-4-yl)-ethyl]-thioureido}-3-(4-methoxyphenyl)-propionyl]-piperidin-4-carboxylat;
1-[(R)-2-(4-Butyryl-4-cyclohexylpiperidin-1-yl)-1-(4-methoxybenzyl)-2-oxoethyl]-3-[2-(1H-imidazol-4-yl)-ethyl]-thioharnstoff;
1-[(R)-2-(4-Cyclohexyl-4-propoxypiperidin-1-yl)-1-(4-methoxybenzyl)-2-oxoethyl]-3-[2-(1H-imidazol-4-yl)-ethyl]-thioharnstoff;
1-[(R)-1-Benzyl-2-(4-cyclohexyl-4-propoxypiperidin-1-yl)-2-oxoethyl]-3-[2-(1H-imidazol-4-yl)-ethyl]-thioharnstoff;
1-[(R)-1-Benzyl-2-(4-cyclohexyl-4-propoxypiperidin-1-yl)-2-oxoethyl]-3-[2-(1H-imidazol-4-yl)-ethyl]-harnstoff;
Ethyl-4-cyclohexyl-1-((R)-3-(4-methoxyphenyl)-2-{3-[2-(3-methyl-3H-imidazol-4-yl)-ethyl]-thioureido}-propionyl)-piperidin-4-carboxylat;
Ethyl-4-cyclohexyl-1-((R)-2-{3-[2-(3-methyl-3H-imidazol-4-yl)-ethyl]-ureido}-3-phenylpropionyl)-piperidin-4-carboxylat;
1-[(R)-2-(4-Cyclohexyl-4-propoxypiperidin-1-yl)-1-(4-methoxybenzyl)-2-oxoethyl]-3-[2-(3-methyl-3H-imidazol-4-yl)-ethyl]-harnstoff;
Ethyl-1-((R)-3-(4-chlorphenyl)-2-{3-[2-(3-methyl-3H-imidazol-4-yl)-ethyl]-ureido}-propionyl)-4-cyclohexylpiperidin-4-carboxylat;
Ethyl-4-cyclohexyl-1-((R)-3-(4-fluorphenyl)-2-{3-[2-(3-methyl-3H-imidazol-4-yl)-ethyl]-ureido}-propionyl)-piperidin-4-carboxylat;
Ethyl-4-cyclohexyl-1-((R)-3-(4-fluorphenyl)-2-{3-[2-(1H-imidazol-4-yl)-ethyl]-ureido}-propionyl)-piperidin-4-carboxylat;
Ethyl-4-cyclohexyl-1-((R)-3-(4-fluorphenyl)-2-{3-[2-(1H-imidazol-4-yl)-ethyl]-thioureido}-propionyl)-piperidin-4-carboxylat;
Ethyl-1-((R)-3-(4-chlorphenyl)-2-{3-[2-(1H-imidazol-4-yl)-ethyl]-thioureido}-propionyl)-4-cyclohexylpiperidin-4-carboxylat;
Ethyl-1-((R)-3-(4-chlorphenyl)-2-{3-[2-(3-methyl-3H-imidazol-4-yl)-ethyl]-thioureido}-propionyl)-4-cyclohexylpiperidin-4-carboxylat;
1-[(R)-2-(4-Cyclohexyl-4-propoxypiperidin-1-yl)-1-(4-fluorbenzyl)-2-oxoethyl]-3-[2-(1H-imidazol-4-yl)-ethyl]-harnstoff;
1-[(R)-1-(4-Chlorbenzyl)-2-(4-cyclohexyl-4-propoxypiperidin-1-yl)-2-oxoethyl]-3-[2-(1H-imidazol-4-yl)-ethyl]-harnstoff;
N-[(R)-2-[3-(4-Hydroxybutoxy)-3-o-tolylazetidin-1-yl]-1-(4-methoxybenzyl)-2-oxoethyl]-3-(1H-imidazol-4-yl)-propionamid;
N-[(R)-2-(3-Cyclopropylmethoxy-3-o-tolylazetidin-1-yl)-1-(4-methoxybenzyl)-2-oxoethyl]-3-(5-methyl-1H-imidazol-4-yl)-propionamid
sowie ihre entsprechenden Salze und Enantiomere.

16. Produkt für die Verwendung nach Anspruch 14, **dadurch gekennzeichnet, dass** die Agonisten des Rezeptors MC1R aus Folgenden ausgewählt sind:
Ethyl-4-cyclohexyl-1-[(R)-2-{3-[2-(1H-imidazol-4-yl)-ethyl]-ureido}-3-(4-methoxyphenyl)-propionyl]-piperidin-4-carboxylat.
N-[(R)-2-[3-(4-Hydroxybutoxy)-3-o-tolylazetidin-1-yl]-1-(4-methoxybenzyl)-2-oxoethyl]-3-(1H-imidazol-4-yl)-propionamid.
N-[(R)-2-(3-Cyclopropylmethoxy-3-o-tolylazetidin-1-yl)-1-(4-methoxybenzyl)-2-oxoethyl]-3-(5-methylimidazol-1H-imidazol-4-yl)-propionamid
sowie den entsprechenden Salzen und Enantiomeren.

17. Agonist des Rezeptors MC1R für eine Verwendung zur Behandlung und/oder Vorbeugung dermatologischer Krankheiten, die mit einer Hypopigmentierung einhergehen, in Verbindung oder in Kombination mit mindestens einem Agonisten von PGF2α-Prostaglandinrezeptoren als Arzneimittel für eine gleichzeitige, getrennte oder zeitlich verteilte Verwendung.

## Claims

1. Combination product comprising at least one prostaglandin PGF2α receptor agonist and at least one MC1R receptor agonist, as a medicament for use simultaneously, separately or spread out over time for the treatment and/or prevention of dermatological conditions linked to hypopigmentation.

2. Product for use according to Claim 1, for the treatment and/or prevention of dermatological conditions linked to hypopigmentation, chosen from vitiligo, albinism, hypomelanosis, depigmentations caused by physical or chemical agents, post-inflammatory hypopigmentations, Sutton's phenomenon or any other hypopigmentary lesions.

3. Product for use according to Claim 2, for the treatment and/or prevention of vitiligo.

4. Product for use according to one of Claims 1 to 3, **characterized in that** the prostaglandin receptor agonist and the MC1R receptor agonist are present in the same composition.

5. Product for use according to one of Claims 1 to 3, **characterized in that** the prostaglandin receptor agonist and the MC1R receptor agonist are present separately from one another in distinct compositions.

6. Product for use according to Claim 5, **characterized in that** the composition comprising the prostaglandin receptor agonist is administered first, and then the composition comprising the MC1R receptor agonist is administered second.

7. Product for use according to one of Claims 4 to 6, **characterized in that** the prostaglandin receptor agonist is present at a concentration of between approximately 0.001% and 1% by weight, relative to the total weight of the composition comprising it.

8. Product for use according to one of Claims 4 to 6, **characterized in that** the MC1R receptor agonist is present at a concentration of between approximately 0.001% and 10% by weight, relative to the total weight of the composition comprising it.

9. Product for use according to Claims 7 and 8, comprising at least one prostaglandin receptor agonist at a concentration of between approximately 0.004% and 0.04% and at least one MC1R receptor agonist at a concentration of between approximately 5% and 10% by weight, relative to the total weight of the composition comprising it, as a medicament for use simultaneously, separately or spread out over time for the treatment and/or prevention of dermatological conditions linked to hypopigmentation.

10. Product for use according to one of Claims 4 to 9, **characterized in that** the composition(s) is (are) administered topically.

11. Product for use according to Claim 10, **characterized in that** the composition(s) is (are) in the form of salves, creams, milks, ointments, powders, impregnated pads, syndets, solutions, gels, sprays, foams, suspensions, sticks, shampoos, washing bases or else suspensions of microspheres, nanospheres or lipid or polymeric vesicles, or polymeric or gelled patches for controlled release.

12. Product for use according to Claim 1, **characterized in that** the prostaglandin receptor agonist is chosen from latanoprost, bimatoprost, travoprost, fluprostenol and unoprostone; preferentially travoprost.

13. Product for use according to one of the preceding claims, **characterized in that** the MC1R receptor agonist is chosen from α, β or γ melanocyte-stimulating hormones (MSH), adrenocorticotropic hormone (ACTH) 1-39, β-lipotropin, β-endorphin and compounds of general formula (I) or (II) below: 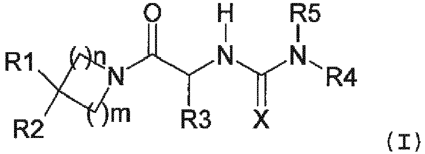 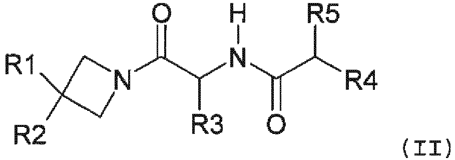 in which, for general formula (I):
R1 represents a hydrogen atom, an aryl, a substituted aryl, an alkyl, a cycloalkyl, a cycloalkylalkyl, or a cycloalkylalkylalkyl;
R2 represents a hydrogen atom, a hydroxyl, a lower alkyl, a substituted lower alkyl, a higher alkyl, a substituted higher alkyl, a cycloalkyl, a cycloalkylalkyl, a lower alkoxy, a substituted lower alkoxy, a higher alkoxy, a substituted higher alkoxy, a cycloalkylalkoxy, an acyloxy, an acyl, an alkoxycarbonyl, a carboxamide, a carboxylic acid, a cyano, or an amino disubstituted with an acyl and an aryl or alkyl;
R3 represents an aralkyl or a substituted aralkyl;
R4 represents a heteroaralkyl or a substituted heteroaralkyl;
R5 represents a hydrogen atom or an alkyl;
X represents an oxygen atom or a sulfur atom;
n and m may be equal to 1 or 2;
and also the corresponding salts and enantiomers;
or in which, for formula (II):
R1 represents an aryl, a substituted aryl or a cycloalkyl;
R2 represents a hydrogen atom, a hydroxyl, a lower alkyl, a substituted lower alkyl, a higher alkyl, a substituted higher alkyl, a cycloalkyl, a cycloalkylalkyl which may be substituted, a lower alkoxy, a substituted lower alkoxy, a higher alkoxy, a substituted higher alkoxy, a cycloalkylalkoxy, or an acyloxy;
R3 represents an aralkyl or a substituted aralkyl;
R4 represents a heteroaralkyl, a substituted heteroaralkyl, a heteroalkyl or a substituted heteroalkyl;
R5 represents a hydrogen atom, a hydroxyl, an amino, an acylamino or a sulfonamide;
and also the corresponding salts and enantiomers,

14. Product for use according to Claim 13, **characterized in that** the MC1R receptor agonist is chosen from the compounds of general formula (II) in which:
R1 represents a cyclopropylmethyl or a 4-hydroxybutyl group;
R2 represents a hydrogen atom or a methyl group.

15. Product for use according to Claim 13, **characterized in that** the MC1R receptor agonist is chosen from:
1-[(S)-2-(4-Butyryl-4-phenylpiperidin-1-yl)-1-(4-methoxybenzyl)-2-oxoethyl]-3-[2-(1H-imidazol-4-yl)-ethyl]urea;
1-[2-(1H-Imidazol-4-yl)ethyl]-3-[1-(4-methoxybenzyl)-2-oxo-2-(4-oxo-1-phenyl-1,3,8-triazaspiro[4.5]-dec-8-yl)ethyl]urea;
1-[2-(4-Cyano-4-phenylpiperidin-1-yl)-1-(4-methoxybenzyl)-2-oxoethyl]-3-[2-(1H-imidazol-4-yl)-ethyl]urea;
1-[2-(1H-Imidazol-4-yl)ethyl]-3-[1-(4-methoxybenzyl)-2-oxo-2-(4-phenylpiperidin-1-yI)ethyl]urea;
1-[2-(1H-Imidazol-4-yl)ethyl]-3-[1-(4-methoxybenzyl)-2-oxo-2-piperidin-1-ylethyl]urea;
Ethyl 4-cyclohexyl-1-[2-{3-[2-(1H-imidazol-4-yl)ethyl]ureido}-3-(4-methoxyphenyl)propionyl]-piperidine-4-carboxylate;
N-{1-[2-{3-[2-(1H-Imidazol-4-yl)ethyl]ureido}-3-(4-methoxyphenyl)propionyl]piperidin-4-yl}-N-phenyl-propionamide;
1-[2-{3-[2-(1H-Imidazol-4-yl)ethyl]ureido}-3-(4-methoxyphenyl)propionyl]-3-phenylazetidin-3-yl butyrate;
Ethyl 1-[2-{3-[2-(1H-imidazol-4-yl)ethyl]ureido}-3-(4-methoxyphenyl)propionyl]piperidine-4-carboxylate;
1-[2-(1H-Imidazol-4-yl)-ethyl]-3-{1-(4-methoxybenzyl)-2-[4-(2-methoxyphenyl)piperidin-1-yl]-2-oxo-ethyl}urea;
1-[2-(3-Butoxy-3-phenylazetidin-1-yl)-1-(4-methoxybenzyl)-2-oxoethyl]-3-[2-(1H-imidazol-4-yl)-ethyl]urea;
Methyl 4-cyclohexyl-1-[2-{3-[2-(1H-imidazol-4-yl)-ethyl]ureido}-3-(4-methoxyphenyl)propionyl]piperidine-4-carboxylamide;
1-[2-(3-Cyclohexanecarbonylazetidin-1-yl)-1-(4-methoxybenzyl)-2-oxoethyl]-3-[2-(1H-imidazol-4-yl)-ethyl]urea;
Ethyl 4-cyclohexyl-1-[2-[3-ethyl-3-[2-(1H-imidazol-4-yl)ethyl]ureido}-3-(4-methoxyphenyl)-propionyl]piperidine-4-carboxylate;
N-Cyclopropyl-N-{1-[2-{3-[2-(1H-imidazol-4-yl)-ethyl]ureido}-3-(4-methoxyphenyl)propionyl]piperidin-4-yl}propionamide;
Ethyl 4-cyclohexyl-1-(2-{3-[2-(1H-imidazol-4-yl)-ethyl]ureido}-3-phenylpropionyl)piperidine-4-carboxylate;
1-[2-(4-Butyryl-4-cyclohexylpiperidin-1-yl)-1-(4-methoxybenzyl)-2-oxoethyl]-3-[2-(3H-imidazol-4-yl)-ethyl] urea;
1-[2-(4-Butoxy-4-cyclohexylpiperidin-1-yl)-1-(4-methoxybenzyl)-2-oxoethyl]-3-[2-(1H-imidazol-4-yl)-ethyl] urea;
Ethyl 4-cyclohexyl-1-(2-{3-[2-(1H-imidazol-4-yl)-ethyl]ureido}-2-phenylacetyl)piperidine-4-carboxylate;
Methyl 4-cyclohexyl-1-[2-{3-[2-(1H-imidazol-4-yl)-ethyl]ureido}-3-(4-methoxyphenyl)propionyl]piperidine-4-carboxylate;
1-[2-(4-Cyclohexyl-4-ethoxypiperidin-1-yl)-1-(4-methoxybenzyl)-2-oxoethyl]-3-[2-(1H-imidazol-4-yl)-ethyl] urea;
1-[2-(4-Acetyl-4-cyclohexylpiperidin-1-yl)-1-(4-methoxybenzyl)-2-oxoethyl]-3-[2-(1H-imidazol-4-yl)-ethyl]urea;
Methyl 4-cyclohexyl-1-(2-{3-[2-(1H-imidazol-4-yl)-ethyl]ureido}-2-phenylacetyl)piperidine-4-carboxylate;
Ethyl 4-ethyl-1-[2-{3-[2-(1H-imidazol-4-yl)ethyl]-ureido}-3-(4-methoxyphenyl)propionyl]piperidine-4-carboxylate;
1-[2-(4-Cyclohexyl-4-propoxypiperidin-1-yl)-1-(4-methoxybenzyl)-2-oxoethyl]-3-[2-(1H-imidazol-4-yl)-ethyl]urea;
4-Cyclohexyl-1-[2-{3-[2-(1H-imidazol-4-yl)ethyl]ureido}-3-(4-methoxyphenyl)propionyl]-piperidine-4-carboxylic acid;
1-[2-(1H-Imidazol-4-yl)ethyl]-3-{1-(4-methoxybenzyl)-2-[3-(2-methylcyclohexyl)-3-propoxyazetidin-1-yl]-2-oxoethyl}urea;
Propyl 4-cyclohexyl-1-[2-{3-[2-(1H-imidazol-4-yl)ethyl]ureido}-3-(4-methoxyphenyl)propionyl]-piperidine-4-carboxylate;
1-[2-(1H-Imidazol-4-yl)ethyl]-3-[1-(4-methoxybenzyl)-2-oxo-2-(3-pentyl-3-phenylazetidin-1-yl)-ethyl]urea;
Ethyl 1-((R)-3-(4-chlorophenyl)-2-{3-[2-(1H-imidazol-4-yl)ethyl]ureido}propionyl)-4-cyclohexyl-piperidine-4-carboxylate;
Ethyl 1-((S)-3-(4-chlorophenyl)-2-{3-[2-(1H-imidazol-4-yl)ethyl]ureido}propionyl)-4-cyclohexyl-piperidine-4-carboxylate;
1-[2-(4-Cyclohexyl-4-propionylpiperidin-1-yl)-1-(4-methoxybenzyl)-2-oxoethyl]-3-[2-(1H-imidazol-4-yl)-ethyl]urea;
1-[2-(4-Cyclohexyl-4-propionylpiperidin-1-yl)-1-(4-methoxybenzyl)-2-oxoethyl]-3-(1H-imidazol-4-yl-methyl)urea;
Ethyl 4-cyclohexyl-1-[(R)-2-{3-[2-(1H-imidazol-4-yl)ethyl]ureido}-3-(4-methoxyphenyl)propionyl]-piperidine-4-carboxylate;
Ethyl 4-cyclopropylmethyl-1-[2-{3-[2-(1H-imidazol-4-yl)ethyl]ureido}-3-(4-methoxyphenyl)propionyl]-piperidine-4-carboxylate;
Propyl 4-cyclohexyl-1-(2-{3-[2-(1H-imidazol-4-yl)-ethyl]ureido}-3-phenylpropionyl)piperidine-4-carboxylate;
Ethyl 4-cyclopentyl-1-(2-{3-[2-(1H-imidazol-4-yl)ethyl]ureido}-3-phenylpropionyl)piperidine-4-carboxylate;
Ethyl 4-cyclopentyl-1-[2-{3-[2-(1H-imidazol-4-yl)ethyl]ureido}-3-(4-methoxyphenyl)propionyl]-piperidine-4-carboxylate;
Ethyl 4-cyclohexyl-1-[(S)-2-{3-[2-(1H-imidazol-4-yl)ethyl]ureido}-3-(4-methoxyphenyl)propionyl]-piperidine-4-carboxylate;
1-[(R)-2-(4-Butyryl-4-cyclohexylpiperidin-1-yl)-1-(4-methoxybenzyl)-2-oxoethyl]-3-[2-(1H-imidazol-4-yl)-ethyl]urea;
1-[(R)-2-(4-Butyryl-4-cyclohexylpiperidin-1-yl)-1-(4-fluorobenzyl)-2-oxoethyl]-3-[2-(1H-imidazol-4-yl)-ethyl] urea;
1-[(R)-1-Benzyl-2-(4-butyryl-4-cyclohexyl-piperidin-1-yl)-2-oxoethyl]-3-[2-(1H-imidazol-4-yl)-ethyl] urea;
1-[(R)-2-(4-Butyryl-4-cyclohexylpiperidin-1-yl)-1-(4-methoxybenzyl)-2-oxoethyl]-3-[2-(3-methyl-3H-imidazol-4-yl)ethyl]urea;
1-[(R)-2-(4-Butyryl-4-cyclohexylpiperidin-1-yl)-1-(4-chlorobenzyl)-2-oxoethyl]-3-[2-(1H-imidazol-4-yl)ethyl]urea;
Ethyl 4-cyclohexyl-1-((R)-3-(3,4-dichlorophenyl)-2-{3-[3-(1H-imidazol-4-yl)propyl]ureido}propionyl)-piperidine-4-carboxylate;
Ethyl 4-cyclohexyl-1-((R)-3-(4-methoxyphenyl)-2-{3-[2-(3-methyl-3H-imidazol-4-yl)ethyl]ureido}-propionyl)piperidine-4-carboxylate;
Ethyl 4-cyclohexyl-1-[(R)-2-{3-[2-(1H-imidazol-4-yl)ethyl]thioureido}-3-(4-methoxyphenyl)propionyl]-piperidine-4-carboxylate;
1-[(R)-2-(4-Butyryl-4-cyclohexylpiperidin-1-yl)-1-(4-methoxybenzyl)-2-oxoethyl]-3-[2-(1H-imidazol-4-yl)-ethyl]thiourea;
1-[(R)-2-(4-Cyclohexyl-4-propoxypiperidin-1-yl)-1-(4-methoxybenzyl)-2-oxoethyl]-3-[2-(1H-imidazol-4-yl)-ethyl]thiourea;
1-[(R)-1-Benzyl-2-(4-cyclohexyl-4-propoxy-piperidin-1-yl)-2-oxoethyl]-3-[2-(1H-imidazol-4-yl)-ethyl]thiourea;
1-[(R)-1-Benzyl-2-(4-cyclohexyl-4-propoxy-piperidin-1-yl)-2-oxoethyl]-3-[2-(1H-imidazol-4-yl)-ethyl]urea;
Ethyl 4-cyclohexyl-1-((R)-3-(4-methoxyphenyl)-2-{3-[2-(3-methyl-3H-imidazol-4-yl)ethyl]thioureido}-propionyl)piperidine-4-carboxylate;
Ethyl 4-cyclohexyl-1-((R)-2-{3-[2-(3-methyl-3H-imidazol-4-yl)ethyl]ureido}-3-phenylpropionyl)-piperidine-4-carboxylate;
1-[(R)-2-(4-Cyclohexyl-4-propoxypiperidin-1-yl)-1-(4-methoxybenzyl)-2-oxoethyl]-3-[2-(3-methyl-3H-imidazol-4-yl)ethyl]urea;
Ethyl 1-((R)-3-(4-chlorophenyl)-2-{3-[2-(3-methyl-3H-imidazol-4-yl)ethyl]ureido}propionyl)-4-cyclohexyl-piperidine-4-carboxylate;
Ethyl 4-cyclohexyl-1-((R)-3-(4-fluorophenyl)-2-{3-[2-(3-methyl-3H-imidazol-4-yl)ethyl]ureido}propionyl)-piperidine-4-carboxylate;
Ethyl 4-cyclohexyl-1-((R)-3-(4-fluorophenyl)-2-{3-[2-(1H-imidazol-4-yl)ethyl]ureido}propionyl)piperidine-4-carboxylate;
Ethyl 4-cyclohexyl-1-((R)-3-(4-fluorophenyl)-2-{3-[2-(1H-imidazol-4-yl)ethyl]thioureido}propionyl)-piperidine-4-carboxylate;
Ethyl 1-((R)-3-(4-chlorophenyl)-2-{3-[2-(1H-imidazol-4-yl)ethyl]thioureido}propionyl)-4-cyclohexyl-piperidine-4-carboxylate;
Ethyl 1-((R)-3-(4-chlorophenyl)-2-{3-[2-(3-methyl-3H-imidazol-4-yl)ethyl]thioureido}propionyl)-4-cyclohexylpiperidine-4-carboxylate;
1-[(R)-2-(4-Cyclohexyl-4-propoxypiperidin-1-yl)-1-(4-fluorobenzyl)-2-oxoethyl]-3-[2-(1H-imidazol-4-yl)-ethyl] urea;
1-[(R)-1-(4-Chlorobenzyl)-2-(4-cyclohexyl-4-propoxypiperidin-1-yl)-2-oxoethyl]-3-[2-(1H-imidazol-4-yl) ethyl] urea;
N-[(R)-2-[3-(4-Hydroxybutoxy)-3-o-tolylazetidin-1-yl]-1-(4-methoxybenzyl)-2-oxoethyl]-3-(-1H-imidazol-4-yl)propionamide
N-[(R)-2-(3-Cyclopropylmethoxy-3-o-tolylazetidin-1-yl)-1-(4-methoxybenzyl)-2-oxoethyl]-3-(5-methyl-1H-imidazol-4-yl)propionamide
and also the respective salts and enantiomers thereof.

16. Product for use according to Claim 14, **characterized in that** the MC1R receptor agonists are chosen from:
Ethyl 4-cyclohexyl-1-[(R)-2-{3-[2-(1H-imidazol-4-yl)ethyl]ureido}-3-(4-methoxyphenyl)propionyl]-piperidine-4-carboxylate;
N-[(R)-2-[3-(4-Hydroxybutoxy)-3-o-tolylazetidin-1-yl]-1-(4-methoxybenzyl)-2-oxoethyl]-3-(-1H-imidazol-4-yl)propionamide;
N-[(R)-2-(3-Cyclopropylmethoxy-3-o-tolylazetidin-1-yl)-1-(4-methoxybenzyl)-2-oxoethyl]-3-(5-methyl-1H-imidazol-4-yl)propionamide
and also the corresponding salts and enantiomers.

17. MC1R receptor agonist for use in the treatment and/or prevention of dermatological conditions linked to hypopigmentation, in association or in combination with at least one prostaglandin PGF2α receptor agonist, as a medicament for use simultaneously, separately or spread out over time.
